# EUROPEAN PATENT APPLICATION

(11) **EP 3 412 666 A1**
(43) Date of publication of application: **12.12.2018**
(21) Application number: 17174818.9
(22) Date of filing: 07.06.2017
(51) Int. Cl.: C07D 471/04

(54) **PROCESS AND INTERMEDIATES FOR THE PREPARATION OF BCL-2 INHIBITORS INCLUDING VENETOCLAX THROUGH REDUCTIVE AMINATION**

(71) Applicant: Albany Molecular Research, Inc., Albany, NY 12203 (US)
(72) Inventor: GREGG, Brian Thomas, Altamont, NY 12009 (US); GEISS, William Bert, Athens, NY 12015 (US); HERR, Robert Jason, Voorheesville, NY 12186 (US)
(74) Representative: ABG Intellectual Property, S.L.

(57) **Abstract**

The invention relates to a process for the preparation of compounds of formula (I), which are useful intermediates in the synthesis of Venetoclax and structurally related compounds, by reductive amination of a compound of formula (II).

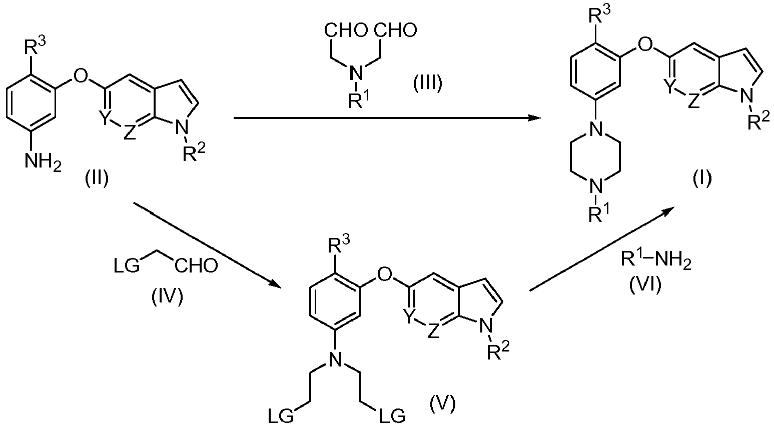

## Description

### Field of the Invention

The invention relates to a process for preparing Venetoclax and structurally related compounds, and to intermediates useful in the preparation of these compounds.

### Background of the Invention

4-(4-{[2-(4-chlorophenyl)-4,4-dimethylcyclohex-1-en-1-yl]methyl}piperazin-1-yl)-N-({3-nitro-4-[(tetrahydro-2H-pyran-4-ylmethyl)amino]phenyl}sulfonyl)-2-(1H-pyrrolo[2,3-b]pyridin-5-yloxy)benzamide (Venetoclax) and other structurally related compounds, are potent and selective Bcl-2 inhibitors having also antitumor activity as apoptosis-inducing agents.

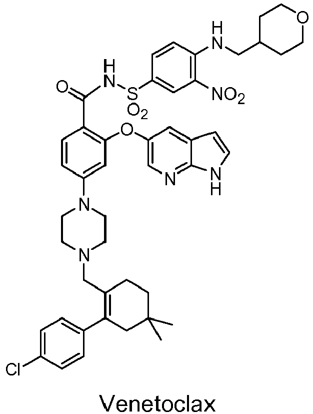

Venetoclax (also known as ABT-199) is a Bcl-2 inhibitor developed by AbbVie. Venetoclax has been disclosed for treating chronic lymphocytic leukemia, lymphoblastic leukemia, follicular lymphoma, a lymphoid malignancy of T cell or B cell origin, myelogenous leukemia, myeloma and breast cancer (US 9174982 B2).

Several methods have been described in the literature for the synthesis of Venetoclax and structurally related apoptosis-inducing agents.

Patent application CN 104370905 A discloses a method for the synthesis of Venetoclax as shown in Scheme 1. S_{N}Ar reaction of methyl 2-fluoro-4-nitrobenzoate with 5-hydroxy-7-azaindole gives rise to intermediate C, which is further hydrogenated to afford aniline D. The piperazine ring is introduced by reaction with dichloride E. Further coupling with intermediate H, hydrolysis of the ester group and coupling with sulfonamide K gives rise to Venetoclax.

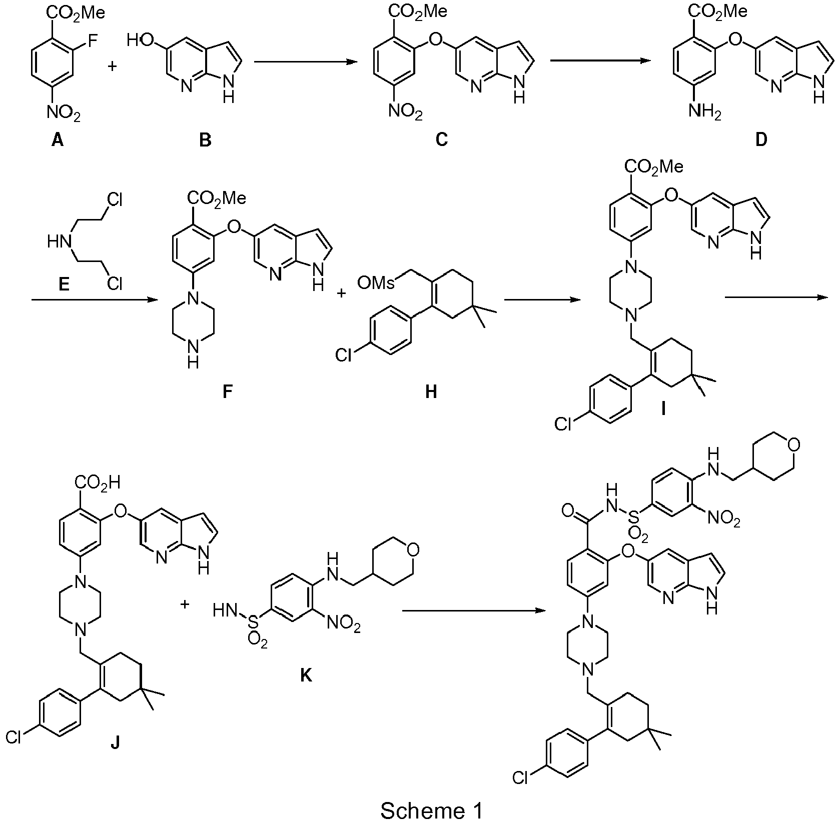

In this synthetic strategy, the piperazine ring is formed by reaction of aniline (D) with the dichloride compound (E). However, when the inventors of the present application tried to obtain compound (F) by reacting compound (D) with dichloride (E) under the reactions conditions disclosed in CN 104370905 A or under usual reactions conditions for this type of transformation, the desired product was not obtained in a satisfactory yield.

It is therefore necessary to develop a new process for obtaining intermediates in the synthesis of Venetoclax and related compounds that overcome all or part of the problems associated with the known processes belonging to the state of the art.

### Summary of the Invention

The invention faces the problem of providing a new process for the preparation of compounds of formula (I), which are useful intermediates in the synthesis of Venetoclax and structurally related compounds. In particular, the inventors have found that compounds of formula (I) can be obtained satisfactorily and under mild reaction conditions through reductive amination of anilines of formula (II).

Thus, in a first aspect, the invention is directed to a process for preparing a compound of formula (I) or a salt or solvate thereof 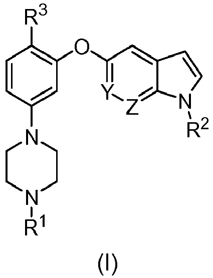 wherein
- R¹: is selected from H, amino protecting group and -CH₂-R⁷;
- R²: is selected from H and amino protecting group;
- R³: is selected from CN and COOR, wherein R is selected from H, C₁-C₆ alkyl, (C₆-C₁₀)aryl(C₁-C₆)alkyl, C₃-C₇cycloalkyl and C₆-C₁₀ aryl;
R⁷ is selected from: 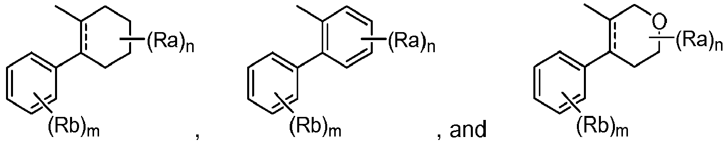 wherein
each Ra is independently selected from OH, CN, halogen and C₁₋C₆ alkyl, each Rb is independently selected from OH, CN, halogen and C₁₋C₆ alkyl, m and n are independently selected from 0, 1, 2 or 3, and
--- is a single or double bond;
- Y: is selected from CH and N; and
- Z: is selected from CH and N;
which comprises reductive amination of a compound of formula (II) or a salt or solvate thereof 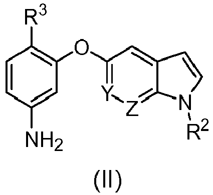 wherein R², R³, Y and Z are as defined above, by:
- reaction with a compound of formula (III) or a salt or solvate thereof 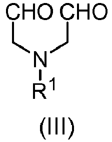 wherein R¹ is selected from H, amino protecting group and -CH₂R⁷, to provide a compound of formula (I) or a salt or solvate thereof;
   or
- reaction with a compound of formula (IV) or a salt or solvate thereof 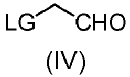 wherein LG is a leaving group, to provide a compound of formula (V) or a salt or solvate thereof 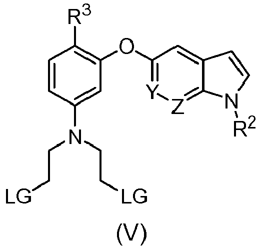 wherein R², R³, Y and Z are as defined above and LG is a leaving group, and
   reaction of a compound of formula (V), or a salt or solvate thereof, with a compound of formula (VI) or a salt or solvate thereof

   R¹-NH₂ (VI)

   wherein R¹ is selected from H, amino protecting group and -CH₂R⁷, to provide a compound of formula (I) or a salt or solvate thereof.

In another aspect, the invention is directed to a compound of formula (V), or a salt or solvate thereof 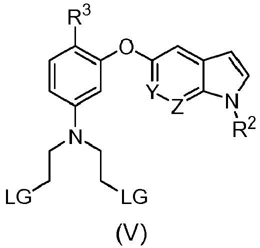 wherein
- R²: is selected from H and amino protecting group;
- R³: is selected from CN and COOR, wherein R is selected from H, C₁-C₆ alkyl, (C₆-C₁₀)aryl(C₁-C₆)alkyl, C₃-C₇cycloalkyl and C₆-C₁₀ aryl;
- Y: is selected from CH and N;
- Z: is selected from CH and N; and
- LG: is a leaving group.

### Detailed Description of the Invention

The term "alkyl" refers to a linear or branched alkane derivative containing from 1 to 6 ("C₁-C₆ alkyl"), preferably from 1 to 3 ("C₁-C₃ alkyl"), carbon atoms and which is bound to the rest of the molecule through a single bond. Illustrative examples of alkyl groups include methyl, ethyl, n-propyl, i-propyl, n-butyl, i-butyl, t-butyl, pentyl, hexyl.

The term "aryl" refers to an aromatic group having between 6 and 10, preferably 6 or 10 carbon atoms, comprising 1 or 2 aromatic nuclei fused to one another. Illustrative examples of aryl groups include phenyl, naphthyl, indenyl, phenanthryl, etc. Preferably, it is phenyl

The term "(C₆-C₁₀)aryl(C₁-C₆)alkyl" refers to an alkyl group as defined above substituted with an aryl group as defined above. Examples of such groups include benzyl, phenylethyl, phenylpropyl, naphthylmethyl, etc. Preferably, it is benzyl.

The term "halogen" refers to bromine, chlorine, iodine or fluorine.

The term "C₃-C₇ cycloalkyl" refers to a radical derived from cycloalkane containing from 3 to 7, preferably from 3 to 6 ("C₃-C₆ cycloalkyl") carbon atoms. Illustrative examples of cycloalkyl groups include cyclopropyl, cyclobutyl, cyclopentyl, cyclohexyl, etc.

The term "heterocyclyl" refers to a saturated or partially unsaturated monocyclic or bicyclic system containing from 3 to 10, preferably 5 to 7, ring atoms containing one or more, specifically one, two, three or four ring heteroatoms independently selected from N, O, and S, and the remaining ring atoms being carbon. Illustrative examples of cycloalkyl groups include tetrahydropyran, morpholine, piperazine, piperidine and [1,4]dioxane.

The term "heteroaryl" refers to an aromatic monocyclic or bicyclic system containing from 3 to 10, preferably 5 to 7, ring atoms containing one or more, specifically one, two, three or four ring heteroatoms independently selected from O, N and S, and the remaining ring atoms being carbon.

The term "amino protecting group" (APG) refers to a group blocking the NH function for subsequent reactions that can be removed under controlled conditions. Amino protecting groups are well known in the art. Illustrative examples of amino protecting groups have been described by Green TW et al. in "Protective Groups in Organic Synthesis", 3rd Edition (1999), Ed. John Wiley & Sons. Virtually any amino protecting group can be used to put the invention into practice. Illustrative, non-limiting examples of APGs include:
- carbamates [-COOR]. R can be selected from C₁-C₆ alkyl, C₁-C₆ alkenyl, C₃-C₇ cycloalkyl, C₆-C₁₀ aryl, (C₆-C₁₀)aryl(C₁C₆)alkyl, 3- to 10-membered heterocyclyl, 3-to 10-membered heteroaryl. Examples of carbamates include methyl carbamate (MOC), t-butyl carbamate (BOC), benzyl carbamate (CBz), 9-fluorenilmetil carbamate (FMOC), trichloroethyl carbamate (TROC), trimethylsilyl carbamate;
- amides [-COR]. R can be selected from C₁-C₆ alkyl, C₁-C₆ alkenyl, C₃-C₇ cycloalkyl, C₆-C₁₀ aryl, (C₆-C₁₀)aryl(C₁-C₆)alkyl, 3- to 10-membered heterocyclyl, 3- to 10-membered heteroaryl. Examples of amides include formamide, acetamide, phenylacetamide, haloacetamide, benzamide, picolinamide;
- amines [-R]. R can be selected from C₁-C₆ alkyl, C₆-C₁₀ aryl and (C₆-C₁₀)aryl(C₁-C₆)alkyl. Examples of amines include methyl amine, tert-butyl amine, benzyl amine, p-methoxybenzyl amine, 3,4-dimethoxybenzyl amine, allyl amine, methoxymethyl amine, triphenylmethyl amine, benzoyl amine, dinitrophenyl amine, p-methoxyphenyl amine; and
- silyl amines [-Si(R)(R')(R")]. R, R' and R" can be independently selected from C₁-C₆ alkyl, C₃-C₇ cycloalkyl, C₆-C₁₀ aryl, C₁-C₆ alkoxy and halogen. Examples of silyl amines include trimethylsilyl amine, triethylsilyl amine, tert-butyldimethylsilyl amine, tert-butyldiphenylsilyl amine, tri-isopropylsilyl amine, triphenylsilyl amine.

The term "leaving group" refers to a functional group or an atom that can be displaced by another functional group in a substitution reaction, such as a nucleophilic substitution reaction. Suitable leaving groups are well known in the art. In a particular embodiment, the leaving group is selected from halogen, C₁₋C₆ alkylsulfonates, C₆-C₁₀ arylsulfonates and C₁C₆alkylC₆-C₁₀arylsulfonates, such as chloro, bromo, iodo, mesylate, triflate, tosylate, nosylate and the like.

As understood in this technical area, there may be a certain degree of substitution in the aforementioned radicals. Therefore, there may be substitution in any of the groups of the present invention. The previous groups can be substituted in one or more available positions with one or more substituents. Said substituents include, for example and in non-limiting sense, C₁₋₆ alkyl, C₃₋₇ cycloalkyl, C₆-C₁₀ aryl, 3- to 10-membered heterocyclyl, 3- to 10-membered heteroaryl, halogen, -CN, NO₂, CF₃, - N(Rₐ)(R_{b}), -OR_{c}, -SR_{d}, -C(O)Rₑ, -C(O)OR_{f}, -C(O)N(R_{g})(Rₕ), -OC(O)Rᵢ; wherein Rₐ, R_{b}, R_{c}, Rd, Rₑ, R_{f}, R_{g}, Rₕ and Rᵢ are independently selected from hydrogen, C₁-C₆ alkyl, C₆-C₁₀ aryl, 3- to 10-membered heterocyclyl, 3- to 10-membered heteroaryl and trifluoromethyl.

The invention also provides "salts" of the compounds described in the present description. By way of illustration, said salts can be acid addition salts, base addition salts or metal salts, and can be synthesized from the parent compounds containing a basic or acid moiety by means of conventional chemical processes known in the art. Such salts are generally prepared, for example, by reacting the free acid or base forms of said compounds with a stoichiometric amount of the suitable base or acid in water or in an organic solvent or in a mixture of the two. Non-aqueous media such as ether, ethyl acetate, ethanol, acetone, isopropanol or acetonitrile are generally preferred. Illustrative examples of said acid addition salts include inorganic acid addition salts such as, for example, hydrochloride, hydrobromide, hydroiodide, sulfate, nitrate, phosphate, etc., organic acid addition salts such as, for example, acetate, maleate, fumarate, citrate, oxalate, succinate, tartrate, malate, mandelate, methanesulfonate, *p-*toluenesulfonate, trifluoroacetate, camphorsulfonate, etc. Illustrative examples of base addition salts include inorganic base salts such as, for example, ammonium salts and organic base salts such as, for example, ethylenediamine, ethanolamine, *N*,*N-*dialkylenethanolamine, triethanolamine, glutamine, amino acid basic salts, etc. Illustrative examples of metal salts include, for example, sodium, potassium, calcium, magnesium, aluminum and lithium salts. In a particular embodiment, the salt is an acid addition salt, such as hydrochloride, hydrobromide, hydroiodide, sulfate, nitrate, phosphate, acetate, maleate, fumarate, citrate, oxalate, succinate, tartrate, malate, mandelate, methanesulfonate, *p*-toluenesulfonate, trifluoroacetate or camphorsulfonate. Preferably, it is selected from HCl, HBr, H₃PO₄, H₂SO₄, MsOH, pTsOH, TFA, citrate and fumarate salt.

Likewise, the compounds described in the present description can be obtained both as free compounds or as solvates (e.g., hydrates, alcoholates, etc.), both forms being included within the scope of the present invention. The solvation methods are generally known in the state of the art. Preferably, the solvate is a hydrate.

The term "organic solvent" includes for example cyclic and acyclic ethers (e.g. Et₂O, iPr₂O, tBu₂O, MeOtBu, 1,4-dioxane, tetrahydrofuran, methyltetrahydrofuran), hydrocarbon solvents (e.g. pentane, hexane, heptane), halogenated solvents (e.g. dichloromethane, chloroform), aromatic solvents (e.g. toluene, xylene), ketones (e.g. acetone, butanone, pentanone, methyl ethyl ketone, ethyl isopropyl ketone), esters (e.g. EtOAc, iPrOAc), nitriles (e.g. acetonitrile, benzonitrile), amides (e.g. DMF, DMA, HMPA), alcohols (e.g. methanol, ethanol, propanol, isopropanol, sec-butanol, t-butanol), sulfoxides (DMSO) and mixtures thereof.

The term "aprotic organic solvent" means any organic solvent that does not yield a proton under the reaction conditions. Suitable examples include, but are not limited to, cyclic and acyclic ethers (e.g. Et₂O, iPr₂O, tBu₂O, MeOtBu, 1,4-dioxane, tetrahydrofuran, methyltetrahydrofuran), hydrocarbon solvents (e.g. pentane, hexane, heptane), halogenated solvents (e.g. dichloromethane, chloroform), aromatic solvents (e.g. toluene, xylene), ketones (e.g. acetone, butanone, pentanone, methyl ethyl ketone, ethyl isopropyl ketone), esters (e.g. EtOAc, iPrOAc), nitriles (e.g. acetonitrile, benzonitrile), amides (e.g. DMF, DMA, HMPA), sulfoxides (DMSO) and mixtures thereof.

### Conversion of a compound of formula (II), or a salt or solvate thereof, into a compound of formula (I), or a salt or solvate thereof ("process of the invention")

In an embodiment of the invention, Y is CH and Z is N in the compound of formula (I).

In an embodiment, R¹ is selected from H and amino protecting group in the compound of formula (I). In another embodiment, R¹ is -CH₂R⁷, wherein R⁷ is selected from: 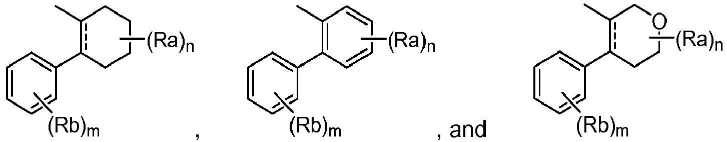 wherein
each Ra is independently selected from OH, CN, halogen and C₁₋C₆ alkyl,
each Rb is independently selected from OH, CN, halogen and C₁₋C₆ alkyl,
m and n are independently selected from 0, 1, 2 or 3, and
--- is a single or double bond.

In an embodiment, R¹ is an amino protecting group in the compound of formula (I). Preferably, it is a carbamate such as BOC. In an embodiment, R² is H in the compound of formula (I). Preferably, R¹ is an amino protecting group and R² is H.

In an embodiment, Y is CH, Z is N and R¹ is an amino protecting group in the compound of formula (I). In an embodiment, Y is CH, Z is N, R¹ is an amino protecting group and R² is H in the compound of formula (I).

In a particular embodiment, R³ is selected from CN and COOR, wherein R is selected from C₁-C₆ alkyl, (C₆-C₁₀)aryl(C₁-C₆)alkyl, C₃-C₇ cycloalkyl and C₆-C₁₀ aryl. In an embodiment, R³ is selected from CN and COOR, wherein R is C₁-C₆ alkyl. In another embodiment, R³ is COOR, wherein R is C₁-C₆ alkyl.

Compounds of formula (II) can be obtained by conventional means known in the art, for example as disclosed in CN 104370905 A.

### Reductive amination

In an embodiment according to the process of the invention, a compound of formula (II), or a salt or solvate thereof, is reacted with a dialdehyde of formula (III), or a salt or solvate thereof, to provide a compound of formula (I), or a salt or solvate thereof.

In another embodiment according to the process of the invention, a compound of formula (II), or a salt or solvate thereof, is reacted with an aldehyde of formula (IV), or a salt or solvate thereof, to provide a compound of formula (V), or a salt or solvate thereof.

This reaction of a compound of formula (II), or a salt or solvate thereof, with a compound of formula (III) or (IV), or a salt or solvate thereof, can be carried out in the presence of a reducing agent. Suitable reducing agents include borohydrides preferably a borohydride such a as NaBH₄, NaCNBH₃, NaBH(OAc)₃, Bu₄NBH₃CN, LiCNBH₃ or 5-ethyl-2-methylpyridine borane (PEMB), preferably NaCNBH₃ or NaBH(OAc)₃.

In an embodiment, the reaction is performed in the presence of an organic solvent. In a particular embodiment, the organic solvent is an alcohol (e.g. methanol, ethanol, propanol, isopropanol, sec-butanol, t-butanol), preferably methanol.

In a particular embodiment, the reaction is performed in the presence of a reducing agent as defined above, preferably NaCNBH₃ or NaBH(OAc)₃, and an organic solvent as defined above, preferably an alcohol.

In a particular embodiment, this reaction is performed at a temperature between -20°C and 120°C, preferably between -20°C and 80°C, more preferably between -10°C and 40°C.

In a particular embodiment, the reducing agent is present in an amount of from 1.0 to 8.0 molar equivalents with respect to the compound of formula (II), preferably from 1.5 to 6.0 molar equivalents, more preferably from 2.0 to 5.0 molar equivalents.

In an embodiment, Y is CH, Z is N and R² is H in the compound of formula (II).

In an embodiment, Y is CH, Z is N, R² is H, and R³ is selected from CN and COOR, wherein R is selected from C₁-C₆ alkyl, (C₆-C₁₀)aryl(C₁-C₆)alkyl, C₃-C₇ cycloalkyl and C₆-C₁₀ aryl, preferably R³ is selected from CN and COOR, wherein R is C₁-C₆ alkyl, more preferably R³ is COOR, wherein R is C₁-C₆ alkyl, in the compound of formula (II).

Preferably, R¹ in the compound of formula (III) is an amino protecting group, preferably a carbamate such as Boc.

In an embodiment, LG in the compound of formula (IV) is selected from chloro, bromo, iodo, mesylate, triflate, tosylate and nosylate. Preferably, it is selected from chloro, bromo and iodo. In an embodiment, LG is chloro.

### Amine Alkylation

A compound of formula (V), or a salt or solvate thereof, can be converted into a compound of formula (I), or a salt or solvate thereof, by reaction with an amine of formula (VI) or a salt or solvate thereof.

Preferably, R¹ in the compound of formula (VI) is an amino protecting group. In a particular embodiment, R¹ in the compound of formula (VI) is Bn.

In a particular embodiment, reaction of a compound of formula (V), or a salt or solvate thereof, with a compound of formula (VI) or a salt or solvate thereof is performed in the presence of a base. Suitable bases include inorganic bases, such a as an alkali metal carbonate or bicarbonate (e.g. Na₂CO₃, K₂CO₃, Cs₂CO₃, NaHCO₃, KHCO₃), an alkali metal phosphate (e.g. Na₃PO₄, K₃PO₄, Na₂HPO₄, K₂HPO₄, NaH₂PO₄, KH₂PO₄), an alkali metal hydroxide (e.g. NaOH, KOH, CsOH), or an alkali metal alkoxide (e.g. NaOMe, KOMe, NaOEt, KOEt, NaOtBu, KOtBu), and tertiary amines (e.g. trimethylamine, triethylamine, diisopropylethylamine (DIPEA), pyridine, 4-dimethylaminopyridine (DMAP), N-methylmorpholine, N,N-dimethylaniline). In a particular embodiment, the base is a tertiary amine, preferably triethylamine or diisopropylethylamine.

Preferably, the reaction is performed in the presence of an organic solvent.

In a particular embodiment, the reaction is performed in the presence of a base, preferably a tertiary amine, and an organic solvent, preferably at a temperature between 20 °C and 120 °C.

In a particular embodiment, LG in the compound of formula (V), or a salt or solvate thereof, is converted into a better leaving group before reacting it with the compound of formula (VI), or a salt or solvate thereof. In an embodiment, a compound of formula (V) wherein LG is Cl, or a salt or solvate thereof, is obtained after reacting a compound of formula (II), or a salt or solvate thereof, with a compound of formula (IV) wherein LG is Cl, and said compound of formula (V) is converted into a compound of formula (V) wherein LG is I. This transformation can be carried out by conventional means known in the art, for example, by reaction with Nal, preferably in acetone.

### Conversion of a compound of formula (I), or a salt or solvate thereof, into a compound of formula (A), or a salt or solvate thereof.

Compounds of formula (I), salts or solvates thereof, are useful intermediates in the synthesis of Venetoclax and related compounds of formula (A).

Therefore, in an embodiment, the process of the invention further comprises converting a compound of formula (I), or a salt or solvate thereof, into a compound of formula (A), or a salt or solvate thereof 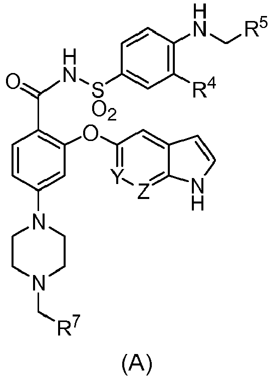 wherein
- Y: is selected from CH and N;
- Z: is selected from CH and N;
- R⁴: is selected from H, NO₂, CN, CF₃ and halogen; and
- R⁵: is selected from H, C₃-C₇ cycloalkyl, and 5- to 10-membered heterocyclyl, optionally substituted by OH, CN, halogen, C₁₋C₆ alkyl and C₃-C₇ cycloalkyl;
R⁷ is selected from: 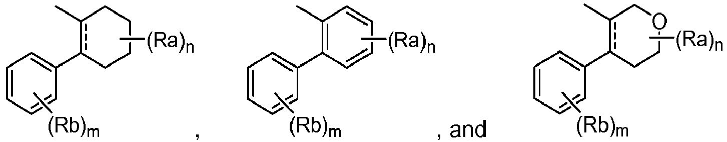 wherein
each Ra is independently selected from OH, CN, halogen and C₁-C₆ alkyl,
each Rb is independently selected from OH, CN, halogen and C₁-C₆ alkyl,
m and n are independently selected from 0, 1, 2 or 3, and
--- is a single or double bond.

In an embodiment, Y is CH.

In an embodiment, Z is N.

In an embodiment, R⁵ is a 5- to 10-membered heterocyclyl, preferably 4-tetrahydropyran.

In an embodiment, R⁴ is NO₂.

In an embodiment, R⁷ is 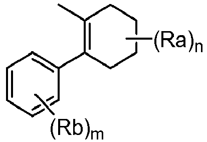 wherein
each Ra is independently selected from C₁₋C₆ alkyl,
each Rb is independently selected from halogen and C₁₋C₆ alkyl, and
m and n are independently selected from 0, 1, 2 or 3.

Preferably, R⁷ is

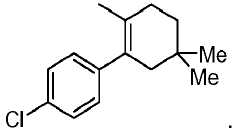

In a particular embodiment, compound of formula (A) is Venetoclax, or a salt or solvate thereof.

Conversion of compounds of formula (I) into compounds of formula (A) has been disclosed in the prior art (CN 104370905 A) or can be performed as disclosed herein below.

### - Route 1.

In a particular embodiment, the process of the invention further comprises converting a compound of formula (I) wherein R¹ is selected from H and amino protecting group, or a salt or solvate thereof, into a compound of formula (A), or a salt or solvate thereof, by a process comprising:
(a) if R³ in the compound of formula (I) herein R¹ is selected from H and amino protecting group, or a salt or solvate thereof, is CN or COOR, wherein R is selected from C₁-C₆ alkyl, (C₆-C₁₀)aryl(C₁-C₆)alkyl, C₃-C₇ cycloalkyl and C₆-C₁₀ aryl, converting it into a compound of formula (Ia) or a salt or solvate thereof 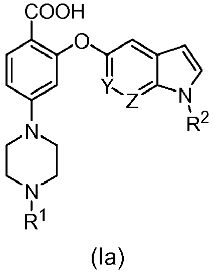 wherein
   - R¹: is selected from H and amino protecting group;
   - R²: is selected from H and amino protecting group;
   - Y: is selected from CH and N; and
   - Z: is selected from CH and N;
(b) reacting a compound of formula (Ia), or a salt or solvate thereof, with a compound of formula (VII) or a salt or solvate thereof 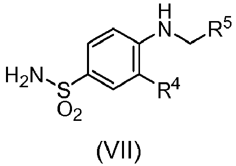 wherein
   - R⁴: is selected from H, NO₂, CN, CF₃ and halogen; and
   - R⁵: is selected from H, C₃-C₇ cycloalkyl, and 5- to 10-membered heterocyclyl, optionally substituted by OH, CN, halogen, C₁-C₆ alkyl and C₃-C₇ cycloalkyl;
   to provide a compound of formula (VIII) or a salt or solvate thereof 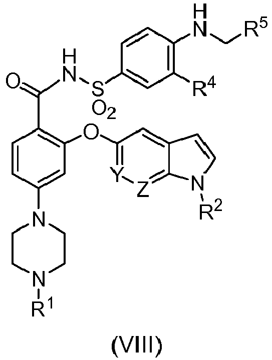 wherein R¹, R², R⁴, R⁵, Y and Z are as defined above;
(c) if R¹ is an amino protecting group, deprotecting it to provide a compound of formula (VIII) wherein R¹ is H, or a salt or solvate thereof;
and
(d) reacting a compound of formula (VIII) wherein R¹ is H, or a salt or solvate thereof, with a compound of formula (IX) or with a compound of formula (X)

   R⁷-CHO (IX)

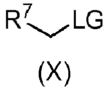

   wherein
   - LG: is a leaving group; and

   - R⁷: is selected from: 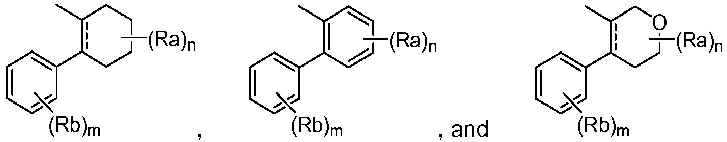 wherein
   each Ra is independently selected from OH, CN, halogen and C₁-C₆ alkyl,
   each Rb is independently selected from OH, CN, halogen and C₁₋C₆ alkyl,
   m and n are independently selected from 0, 1, 2 or 3, and
   --- is a single or double bond;
to provide a compound of formula (A), or a salt or solvate thereof.

This process can comprise, if necessary, one or more of the following steps in any order:
- cleavage of one or two of the amino protecting groups (R¹ and R²), and/or
- protection of one or two of the amino groups (R¹ and R²).

Any of these steps can be carried out either before or after any of steps (a), (b), (c) and/or (d). Protection/cleavage of the amino protecting group can be carried out by any conventional means known in the art (e.g. T.H. Greene and P.G.M. Wuts, Protective Groups in Organic Synthesis, 4th edition, John Wiley & Sons, 2007) and as disclosed herein for R¹.

### Step (a)

In a particular embodiment, R³ in the starting compound of formula (I) wherein R¹ is selected from H and amino protecting group, or a salt or solvate thereof, is CN or COOR, wherein R is selected from C₁-C₆ alkyl, (C₆-C₁₀)aryl(C₁-C₆)alkyl, C₃-C₇ cycloalkyl and C₆-C₁₀ aryl. Preferably, it is COOR, wherein R is selected from C₁-C₆ alkyl and (C₆-C₁₀)aryl(C₁-C₆)alkyl. More preferably, it is COOR, wherein R is selected from C₁-C₆ alkyl, such as COOMe.

In a particular embodiment, Y is CH, Z is N, R¹ is an amino protecting group and R² is H in the starting compound of formula (I).

Reaction conditions for the conversion of a compound of formula (I) wherein R³ is CN or COOR, wherein R is selected from C₁-C₆ alkyl, (C₆-C₁₀)aryl(C₁-C₆)alkyl, C₃-C₇ cycloalkyl and C₆-C₁₀ aryl, or a salt or solvate thereof, into a compound of formula (Ia), or a salt or solvate thereof, are well-known for the skilled person in the art.

In a particular embodiment, when R³ is CN in the starting compound of formula (I), or a salt or solvate thereof, it can be converted into a compound of formula (Ia), or a salt or solvate thereof, by acid or basic hydrolysis. In a particular embodiment, the conversion is carried out by treatment with an acid or a base under heat. Suitable acids include acetic acid, trifluoroacetic acid, methanesulfonic acid, trifluoromethanesulfonic acid, hydrochloric acid, hydrobromic acid, hydrofluoric acid, perchloric acid, sulfuric acid, nitric acid, phosphoric acid, formic acid, propionic acid, butyric acid, malic acid, citric acid, benzoic acid, p-toluenesulfonic acid, oxalic acid and succinic acid, preferably hydrochloric acid, hydrobromic acid and sulfuric acid. Suitable bases include alkali metal alkoxides and alkali metal hydroxides, such as NaOEt, NaOMe, NaOtBu, KOEt, KOMe, KOtBu, NaOH, LiOH, KOH, CsOH, preferably NaOH, KOH and NaOMe, more preferably NaOH. In an embodiment, the reaction is carried out in the presence of water and an organic solvent, preferably an alcohol (e.g. methanol, ethanol, n-propanol, i-propanol, n-butanol, t-butanol, 2-butanol, 2-pentanol, 2-hexanol, 2-octanol, ethylene glycol). In an embodiment, the reaction is carried out at a temperature between 40°C and the reflux temperature of the solvent, preferably between 40°C and 120°C, more preferably between 60°C and 110°C, more preferably between 80°C and 100°C. In an embodiment, the reaction is carried out in the presence of an acid selected from hydrochloric acid, hydrobromic acid and sulfuric acid; water and an organic solvent, preferably an alcohol; at a temperature between 60°C and 110°C. In another embodiment, the reaction is carried out in the presence of a base selected from NaOH, KOH and NaOMe; water and an organic solvent, preferably an alcohol; at a temperature between 60°C and 110°C.

In another embodiment, when R³ is CN in the starting compound of formula (I), or a salt or solvate thereof, it can be converted into a compound of formula (Ia), or a salt or solvate thereof, by reduction of the nitrile to aldehyde followed by oxidation of the aldehyde group to carboxylic acid. Conditions for the reduction of the nitrile to aldehyde and oxidation of the aldehyde to carboxylic acid are well-known for the skilled person. In a particular embodiment, reduction of the nitrile to aldehyde is performed in the presence of an aluminium hydride, such as diisobutylaluminum hydride. In an embodiment, oxidation of the aldehyde to carboxylic acid is performed in the presence of an oxidizing agent, such as KMnO₄, K₂Cr₂O_{7,} Ag₂O, H₂O₂, PCC or oxone. Preferably these reactions are performed in the presence of an organic solvent.

In a particular embodiment, when R³ is COOR, wherein R is selected from C₁-C₆ alkyl, (C₆-C₁₀)aryl(C₁-C₆)alkyl, C₃-C₇ cycloalkyl and C₆-C₁₀ aryl, in the starting compound of formula (I), or a salt or solvate thereof, it can be converted into a compound of formula (Ia), or a salt or solvate thereof, by acid or basic hydrolysis. Suitable acids include acetic acid, trifluoroacetic acid, methanesulfonic acid, trifluoromethanesulfonic acid, hydrochloric acid, hydrobromic acid, hydrofluoric acid, perchloric acid, sulfuric acid, nitric acid, phosphoric acid, formic acid, propionic acid, butyric acid, malic acid, citric acid, benzoic acid, p-toluenesulfonic acid, oxalic acid and succinic acid, preferably hydrochloric acid, hydrobromic acid and sulfuric acid. Suitable bases include alkali metal alkoxides and alkali metal hydroxides, such as NaOEt, NaOMe, NaOtBu, KOEt, KOMe, KOtBu, NaOH, LiOH, KOH, CsOH, preferably NaOH, KOH and NaOMe, more preferably NaOH. In an embodiment, the reaction is carried out in the presence of water and an organic solvent, preferably an alcohol (e.g. methanol, ethanol, n-propanol, i-propanol, n-butanol, t-butanol, 2-butanol, 2-pentanol, 2-hexanol, 2-octanol, ethylene glycol). In an embodiment, the reaction is carried out at a temperature between 0°C and the reflux temperature of the solvent, preferably between 0°C and 100°C, more preferably between 10°C and 80°C, more preferably between 20°C and 50°C. In an embodiment, the reaction is carried out in the presence of an acid selected from hydrochloric acid, hydrobromic acid and sulfuric acid; water and an organic solvent, preferably an alcohol. In another embodiment, the reaction is carried out in the presence of a base selected from NaOH, KOH and NaOMe; water and an organic solvent, preferably an alcohol.

### Step (b)

In an embodiment, R⁵ is 4-tetrahydropyran and R⁴ is NO₂ in the compound of formula (VII), or a salt or solvate thereof.

In an embodiment, R¹ in the compounds of formula (Ia) and (VIII) is an amino protecting group. In a particular embodiment, R¹ and R² in the compounds of formula (Ia) and (VIII) are independently selected from an amino protecting group. In another embodiment, R¹ is an amino protecting group and R² is H in the compounds of formula (Ia) and (VIII).

Preferably, R⁵ is 4-tetrahydropyran, R⁴ is NO₂, R¹ is an amino protecting group and R² is H in the compounds of formula (Ia) and (VIII).

In a particular embodiment, step (b) is performed in the presence of a coupling agent and a base. Suitable coupling agents are known to those skilled in the art and include 1-ethyl-3-(3-dimethylaminopropyl)carbodiimide (EDAC), N,N'-dicyclohexylcarbodimide (DCC), benzotriazol-1-yl-oxy-tris(dimethylamino) phosphonium hexafluorophosphate (BOP), EDCI, 2-propanephosphonic acid anhydride (T3P), HATU, O-benzotriazol-1-yl-N,N,N,N-tetramethyluronium hexafluorophosphate (HBTU) and bromo-tripyrrolidino-phosphonium hexafluorophosphates, preferably EDAC. Suitable bases include tertiary amines such as trimethylamine, triethylamine, diisopropylethylamine (DIPEA), pyridine, 4-dimethylaminopyridine (DMAP), N-methylmorpholine, preferably triethylamine, DMAP and mixtures thereof.

In a particular embodiment the reaction is performed in the presence of an organic solvent, preferably tetrahydrofuran, methyltetrahydrofuran, dichloromethane or mixtures thereof. In a particular embodiment, the organic solvent is dichloromethane.

In a particular embodiment, this reaction is performed at a temperature between 0°C and 100°C, preferably between 10°C and 60°C, more preferably between 10°C and 30°C.

In an embodiment, step (b) is performed in the presence of EDAC, DMAP and dichloromethane, preferably at a temperature between 10°C and 60°C.

### Step (c)

If a compound of formula (VIII) wherein R¹ is an amino protecting group, or a salt or solvate thereof, is obtained after step (b), then it will be necessary to cleave the amino protecting group to obtain a compound of formula (VIII) wherein R¹ is H, or a salt or solvate thereof, for its further reaction with a compound of formula (IX) or (X).

Cleavage of the amino protecting group can be carried out by any conventional means known in the art (e.g. T.H. Greene and P.G.M. Wuts, Protective Groups in Organic Synthesis, 4th edition, John Wiley & Sons, 2007).

For instance, when the amino protecting group in R¹ is a carbamate (R¹=COOR) it can be easily deprotected by acid or basic hydrolysis according to well-stablished procedures of the state of the art. Suitable acids include acetic acid, trifluoroacetic acid, methanesulfonic acid, trifluoromethanesulfonic acid, hydrochloric acid, hydrobromic acid, hydrofluoric acid, perchloric acid, sulfuric acid, nitric acid, phosphoric acid, formic acid, propionic acid, butyric acid, malic acid, citric acid, fumaric acid, benzoic acid, TFA, MsOH, pTsOH, oxalic acid and succinic acid, preferably HCl, HBr, H₃PO₄, H₂SO₄, MsOH, pTsOH, TFA, citric acid and fumaric acid. Suitable bases include alkali metal carbonates, alkali metal phosphates, alkali metal alkoxides and alkali metal hydroxides. In an embodiment, the reaction is carried out in the presence of water and an organic solvent, preferably an alcohol (e.g. methanol, ethanol, n-propanol, i-propanol, n-butanol, t-butanol, 2-butanol, 2-pentanol, 2-hexanol, 2-octanol, ethylene glycol). In an embodiment, the reaction is carried out at a temperature between 10°C and the reflux temperature of the solvent, preferably between 30°C and 70°C, more preferably between 40°C and 60°C. In an embodiment, the reaction is performed in the presence of an acid selected from HCl, HBr, H₃PO₄, H₂SO₄, MsOH, pTsOH, TFA, citrate and fumarate, preferably HCI; water and an alcohol, preferably methanol; at a temperature between 30°C and 70°C, preferably between 40°C and 60°C.

When the amino protecting group in R¹ is an amide (R¹=COR) it can deprotected by acid or basic hydrolysis, preferably under heat. Suitable acids include acetic acid, trifluoroacetic acid, methanesulfonic acid, trifluoromethanesulfonic acid, hydrochloric acid, hydrobromic acid, hydrofluoric acid, perchloric acid, sulfuric acid, nitric acid, phosphoric acid. Suitable bases include alkali metal carbonates, alkali metal phosphates, alkali metal alkoxides and alkali metal hydroxides. The reaction can be carried out at a temperature between 20°C and 120°C, preferably between 50°C and 110°C, more preferably between 60°C and 110°C, and can be performed in the presence of an organic solvent, water and mixtures thereof.

When the amino protecting group in R¹ is an alkyl, aryl or arylalkyl amine (R¹=R) it can be deprotected by treatment with an acid, a base, an oxidant, a reductant, by hydrogenolysis (for aryl or arylalkyl amines), etc.

When the amino protecting group in R¹ is a silyl amine (R¹= Si(R)(R')(R")) it can be deprotected by the use of fluoride reagents such as fluoride salts or HF, acid media, oxidizing media, etc.

In a preferred embodiment, step (c) is carried out by treatment with an acid, to provide a compound of formula (VIII') or a solvate thereof 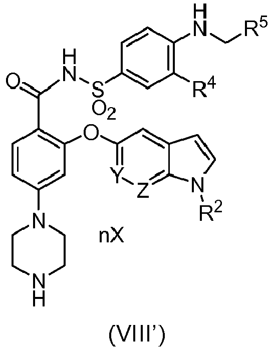 wherein R², R⁴, R⁵, Y and Z are as defined above for the compound of formula (VIII), n is selected from 1 and 2, and X is an acid.

In a particular embodiment, Y is CH, Z is N, R⁵ is 4-tetrahydropyran, R⁴ is NO₂, and R² is H. Preferably X is selected from Cl, HBr, H₃PO₄, H₂SO₄, MsOH, pTsOH, TFA, citrate and fumarate, more preferably X is HCl.

According to an embodiment of the invention, treatment of a compound of formula (VIII), or a solvate thereof, with an acid is performed in the presence of an organic solvent, preferably an alcohol such as methanol, ethanol, propanol, isopropanol or t-butanol.

In an embodiment, the acid (an so X) is selected from hydrochloric, hydrobromic, hydroiodic, sulfuric, nitric, phosphoric, acetic, maleic, fumaric, citric, oxalic, succinic, tartraric, malic, mandelic, methanesulfonic, *p*-toluenesulfonic, trifluoroacetic or camphorsulfonic acid. Preferably, it is selected from HCl, HBr, H₃PO₄, H₂SO₄, MsOH, pTsOH, TFA, citric acid and fumaric acid, more preferably HCl.

In a particular embodiment, the reaction is carried out in the presence of an acid, preferably HCl, HBr, H₃PO₄, H₂SO₄, MsOH, pTsOH, TFA, citric acid and fumaric acid, more preferably HCl, and an alcohol, preferably methanol. In an embodiment, the reaction is carried out at a temperature between room temperature and the reflux temperature of the solvent, preferably at a temperature between 30°C and 70°C, more preferably between 40°C and 60°C.

In a preferred embodiment, R¹ in the compound of formula (VIII) is an acid labile amino protecting group, so that when the compound of formula (VIII), or a solvate thereof, is treated with an acid, both the amino protecting group is cleaved and the acid addition salt is formed thus giving rise a compound of formula (VIII'), or a solvate thereof. Preferably, R¹ in the compound of formula (VII) is a carbamate.

The inventors have observed that intermediates of formula (VIII') are obtained as a crystalline solid form. In particular, they have observed that salts of formula (VIII') crystallize from the reaction mixture and can be isolated directly. Additionally, in this way, minor impurities formed along the synthetic route are purged. Finally, since they are crystalline they have favorable stability for storage purposes.

### Step (d)

Preferably, R⁷ in the compounds of formula (IX) and (X) is

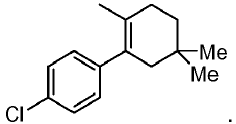

In a preferred embodiment, the compound of formula (VIII) wherein R¹ is H is a salt thereof of formula (VIII'), or a solvate thereof.

In a particular embodiment, a compound of formula (VIII) wherein R¹ is H, or a salt or solvate thereof, is converted into a compound of formula (A), or a salt or solvate thereof, by treatment with a compound of formula (IX) (reductive amination).

In a particular embodiment reaction of the compound of formula (VIII) wherein R¹ is H, or a salt or solvate thereof, with a compound of formula (IX) is performed in the presence of a reducing agent. Suitable reducing agents include borohydrides preferably a borohydride such a as NaBH₄, NaCNBH₃, NaBH(OAc)₃, Bu₄NBH₃CN, LiCNBH₃, preferably NaBH(OAc)₃.

In a particular embodiment reaction of the compound of formula (VIII) wherein R¹ is H, or a salt or solvate thereof, with a compound of formula (IX) is performed in the presence of a base. Suitable bases include tertiary amines such as trimethylamine, triethylamine, diisopropylethylamine (DIPEA), pyridine, 4-dimethylaminopyridine (DMAP), N-methylmorpholine, preferably DIPEA and triethylamine.

In an embodiment, the reaction is performed in the presence of a reducing agent, as defined above, and a base, as defined above. Preferably, in the presence of NaBH(OAc)₃ and DIPEA or triethylamine.

In an embodiment reaction of the compound of formula (VIII) wherein R¹ is H, or a salt or solvate thereof, with a compound of formula (IX) is performed in the presence of an organic solvent, preferably a cyclic or acyclic ether (e.g. Et₂O, iPr₂O, tBuOMe, 1,4-dioxane, tetrahydrofuran, methyltetrahydrofuran), a halogenated solvent (e.g. dichloromethane, chloroform, dichloroethane), and mixtures thereof. Preferably the organic solvent is dichloromethane, dichloroethane, tetrahydrofuran, methyltetrahydrofuran or mixtures thereof. In a particular embodiment, the organic solvent is selected from dichloromethane, tetrahydrofuran and mixtures thereof.

In a particular embodiment, the reaction is performed in the presence of a reducing agent as defined above, preferably NaBH(OAc)₃, and an organic solvent as defined above.

In a particular embodiment, this reaction is performed at a temperature between 0°C and 120°C, preferably between 10°C and 80°C, more preferably between 15°C and 50°C.

In an embodiment, the reaction is performed in the presence of a reducing agent, an organic solvent and optionally a base, preferably at a temperature between 15°C and 50°C. In a particular embodiment, the reaction is performed in the presence of NaBH(OAc)₃, an organic solvent and optionally a base, preferably at a temperature between 15°C and 50°C.

In another embodiment of the invention, a compound of formula (VIII) wherein R¹ is H, or a salt or solvate thereof, is converted into a compound of formula (A), or a salt or solvate thereof, by treatment with a compound of formula (X).

In a particular embodiment, LG in the compound of formula (X) is selected from halogen, C₁-C₆ alkylsulfonates, C₆-C₁₀ arylsulfonates and C₁C₆alkylC₆-C₁₀arylsulfonates; preferably it is selected from chloro, bromo, iodo, mesylate, triflate, tosylate and nosylate.

In a particular embodiment reaction of the compound of formula (VIII) wherein R¹ is H, or a salt or solvate thereof, with a compound of formula (X) is performed in the presence of a base. Suitable bases include alkali metal carbonates or bicarbonates (e.g. Na₂CO₃, K₂CO₃, Cs₂CO₃, NaHCO₃, KHCO₃), an alkali metal phosphates (e.g. Na₃PO₄, K₃PO₄, Na₂HPO₄, K₂HPO₄, NaH₂PO₄, KH₂PO₄), alkali metal alkoxides (e.g. NaOMe, KOMe, NaOEt, KOEt, NaOtBu, KOtBu), alkali metal hydroxides (e.g. NaOH, LiOH, KOH, CsOH) and tertiary amines (e.g. trimethylamine, triethylamine, DIPEA, pyridine, DMAP, N-methylmorpholine).

In an embodiment reaction of the compound of formula (VIII) wherein R¹ is H, or a salt or solvate thereof, with a compound of formula (X) is performed in the presence of an organic solvent, preferably an aprotic organic solvent.

In a particular embodiment, this reaction is performed at a temperature between 10°C and 120°C, preferably between 20°C and 100°C, more preferably between 30°C and 80°C.

In an embodiment, the reaction is performed in the presence of a base and an organic solvent, preferably at a temperature between 20°C and 100°C.

### - Route 2.

In a particular embodiment, the process of the invention further comprises converting a compound of formula (I) wherein R¹ is selected from H and amino protecting group, or a salt or solvate thereof, into a compound of formula (A), or a salt or solvate thereof, by a process comprising:
(a') if R¹ in the compound of formula (I) is an amino protecting group, deprotecting it to provide a compound of formula (Ib), or a salt or solvate thereof, 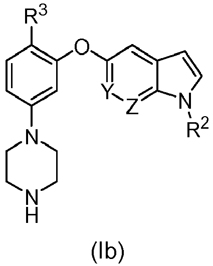 wherein
   - R²: is selected from H and amino protecting group;
   - R³: is selected from CN and COOR, wherein R is selected from H, C₁-C₆ alkyl, (C₆-C₁₀)aryl(C₁-C₆)alkyl, C₃-C₇ cycloalkyl and C₆-C₁₀ aryl;
   - Y: is selected from CH and N; and
   - Z: is selected from CH and N;
(b') reacting a compound of formula (Ib), or a salt or solvate thereof, with a compound of formula (IX) or with a compound of formula (X)

   R⁷-CHO (IX)

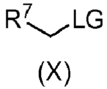

   wherein
   - LG: is a leaving group; and
   - R⁷: is selected from: 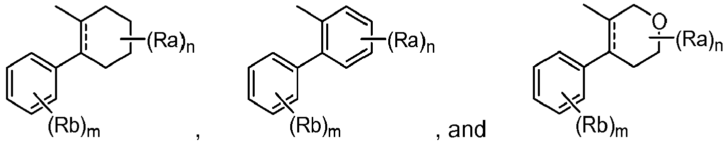 wherein
   each Ra is independently selected from OH, CN, halogen and C₁-C₆ alkyl,
   each Rb is independently selected from OH, CN, halogen and C₁-C₆ alkyl,
   m and n are independently selected from 0, 1, 2 or 3, and
   --- is a single or double bond;
   to provide a compound of formula (Ic) or a salt or solvate thereof 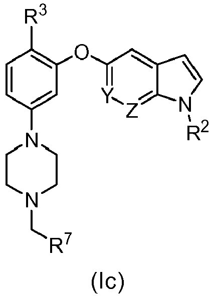 wherein R², R³, R⁷, Y and Z are as defined above;
(c') if R³ in the compound of formula (Ic), or a salt or solvate thereof, is CN or COOR, wherein R is selected from C₁-C₆ alkyl, (C₆-C₁₀)aryl(C₁-C₆)alkyl, C₃-C₇ cycloalkyl and C₆-C₁₀ aryl, converting it into a compound of formula (Id) or a salt or solvate thereof 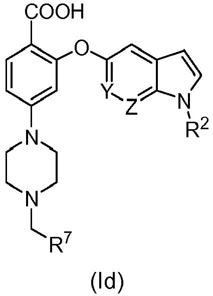 wherein R², R⁷, Y and Z are as defined above; and
(d') reacting a compound of formula (Id), or a salt or solvate thereof, with a compound of formula (VII) or a salt or solvate thereof 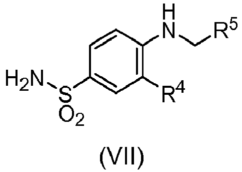 wherein
   - R⁴: is selected from H, NO₂, CN, CF₃ and halogen; and
   - R⁵: is selected from H, C₃-C₇ cycloalkyl, and 5- to 10-membered heterocyclyl, optionally substituted by OH, CN, halogen, C₁-C₆ alkyl and C₃-C₇ cycloalkyl;
to provide a compound of formula (A), or a salt or solvate thereof.

This process can comprise, if necessary, one or more of the following steps in any order:
- cleavage of one or two of the amino protecting groups (R¹ and R²), and/or
- protection of one or two of the amino groups (R¹ and R²).

Any of these steps can be carried out either before or after any of steps (a'), (b'), (c') and/or (d'). Protection/cleavage of the amino protecting group can be carried out by any conventional means known in the art (e.g. T.H. Greene and P.G.M. Wuts, Protective Groups in Organic Synthesis, 4th edition, John Wiley & Sons, 2007) and as disclosed herein for R¹.

Suitable and preferred conditions for step (a') are as disclosed herein for step (c) in synthetic route 1.

In a particular embodiment, R¹ in the starting compound of formula (I) is an acid labile amino protecting group, e.g. a carbamate, and step (a') is carried out in the presence of an acid (as disclosed herein for step (c) in synthetic route 1) so that an acid addition salt of the compound of formula (Ib), or a solvate thereof, is obtained and used in subsequent step (b').

Suitable and preferred conditions for step (b'), as well as particular and preferred values for R⁷ and LG in the compounds of formula (IX) and (X), are as disclosed herein for step (d) in synthetic route 1.

In an embodiment, Y is CH and Z is N in the compounds of formula (Ib) and (Ic). Preferably, Y is CH, Z is N and R² is H in the compounds of formula (Ib) and (Ic).

In a particular embodiment, R³ in the compounds of formula (Ib) and (Ic) is CN or COOR, wherein R is selected from C₁-C₆ alkyl, (C₆-C₁₀)aryl(C₁C₆)alkyl, C₃-C₇ cycloalkyl and C₆-C₁₀ aryl. Preferably, it is COOR, wherein R is selected from C₁-C₆ alkyl and (C₆-C₁₀)aryl(C₁-C₆)alkyl. More preferably, it is COOR, wherein R is selected from C₁₋C₆ alkyl, such as COOMe.

Suitable and preferred conditions for step (c') are as disclosed herein for step (a) in synthetic route 1.

Suitable and preferred conditions for step (d'), as well as particular and preferred values for R², R⁴, R⁵ and R⁷, are as disclosed herein for step (b) in synthetic route 1.

### - Route 3.

In a particular embodiment, the process of the invention further comprises converting a compound of formula (I) wherein R¹ is -CH₂-R⁷ (i.e. a compound of formula (Ic)) or a salt or solvate thereof, into a compound of formula (A), or a salt or solvate thereof, by a process comprising:
(a") if R³ in the compound of formula (Ic), or a salt or solvate thereof, is CN or COOR, wherein R is selected from C₁-C₆ alkyl, (C₆-C₁₀)aryl(C₁-C₆)alkyl, C₃-C₇ cycloalkyl and C₆-C₁₀ aryl, converting it into a compound of formula (Id) or a salt or solvate thereof 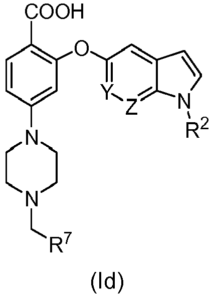 wherein R², R⁷, Y and Z are as defined above; and
(b") reacting a compound of formula (Id), or a salt or solvate thereof, with a compound of formula (VII) or a salt or solvate thereof 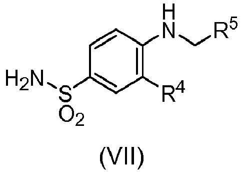 wherein
   - R⁴: is selected from H, NO₂, CN, CF₃ and halogen; and
   - R⁵: is selected from H, C₃-C₇ cycloalkyl, and 5- to 10-membered heterocyclyl, optionally substituted by OH, CN, halogen, C₁-C₆ alkyl and C₃-C₇ cycloalkyl;
to provide a compound of formula (A), or a salt or solvate thereof.

This process can comprise, if necessary, one or more of the following steps in any order:
- cleavage of the amino protecting groups (R²), and/or
- protection of the amino groups (R²).

Any of these steps can be carried out either before or after any of steps (a") and/or (b"). Protection/cleavage of the amino protecting group can be carried out by any conventional means known in the art (e.g. T.H. Greene and P.G.M. Wuts, Protective Groups in Organic Synthesis, 4th edition, John Wiley & Sons, 2007) and as disclosed herein for R¹.

Suitable and preferred conditions for step (a") are as disclosed herein for step (a) in synthetic route 1.

In an embodiment, Y is CH and Z is N in the compounds of formula (Ic) and (Id). Preferably, Y is CH, Z is N and R² is H in the compounds of formula (Ic) and (Id).

In a particular embodiment, R³ in the starting compound of formula (Ic) is CN or COOR, wherein R is selected from C₁-C₆ alkyl, (C₆-C₁₀)aryl(C₁-C₆)alkyl, C₃-C₇ cycloalkyl and C₆-C₁₀ aryl. Preferably, it is COOR, wherein R is selected from C₁-C₆ alkyl and (C₆-C₁₀)aryl(C₁-C₆)alkyl. More preferably, it is COOR, wherein R is selected from C₁-C₆ alkyl, such as COOMe.

Suitable and preferred conditions for step (b"), as well as particular and preferred values for R², R⁴, R⁵ and R⁷, are as disclosed herein for step (b) in synthetic route 1.

### Intermediate compounds

Compounds of formula (V), and salts or solvates thereof, are useful intermediates in the preparation of compounds of formula (I) and, therefore, in the synthesis of Venetoclax and structurally related compounds of formula (A).

In another aspect, the invention is directed to a compound of formula (V) or a salt or solvate thereof 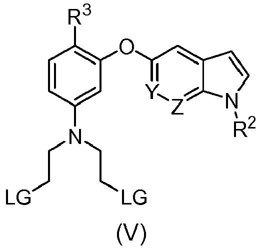 wherein
- R²: is selected from H and amino protecting group;
- R³: is selected from CN and COOR, wherein R is selected from H, C₁₋C₆ alkyl, (C₆-C₁₀)aryl(C₁C₆)alkyl, C₃-C₇ cycloalkyl and C₆-C₁₀ aryl;
- Y: is selected from CH and N;
- Z: is selected from CH and N; and
- LG: is a leaving group.

In an embodiment of the invention, Y is CH and Z is N.

In an embodiment, R² is H.

Preferably, Y is CH, Z is N and R² is H.

In a particular embodiment, R³ is selected from CN and COOR, wherein R is selected from C₁-C₆ alkyl, (C₆-C₁₀)aryl(C₁-C₆)alkyl, C₃-C₇ cycloalkyl and C₆-C₁₀ aryl. In an embodiment, R³ is selected from CN and COOR, wherein R is C₁-C₆ alkyl. In another embodiment, R³ is COOR, wherein R is C₁-C₆ alkyl.

In an embodiment, LG is selected from chloro, bromo, iodo, mesylate, triflate, tosylate and nosylate, preferably it is selected from chloro, bromo and iodo.

In a particular embodiment, Y is CH, Z is N, R² is H and R³ is COOR, wherein R is C₁-C₆ alkyl.

In a particular embodiment, the compound of formula (V) is a compound of the following formula 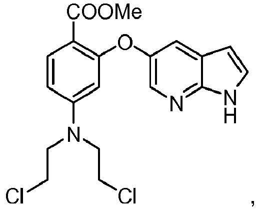 or a salt or solvate thereof.

It should be understood that the scope of the present disclosure includes all the possible combinations of embodiments disclosed herein.

### EXAMPLES

### Example 1: Preparation of tert-butyl 3,4-dihydroxypyrrolidine-1-carboxylate (61)

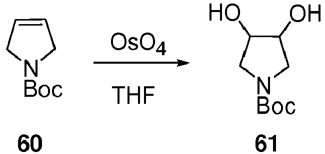

*tert*-Butyl 2,5-dihydro-1H-pyrrole-1-carboxylate (60, 1.00 g, 5.90 mmol) was dissolved in THF (20 mL) and treated with 0.1 M osmium tetroxide in toluene (0.6 mL, 0.06 mmol) followed by the addition of *N*-methylmorpholine *N*-oxide (0.73 g, 6.23 mmol). The reaction was stirred at room temperature for 22 h and was then quenched by the addition of aqueous sodium bisulfite (20 mL). The layers were separated and the aqueous layer was extracted with ethyl acetate (50 mL). The combined organic layers were dried with sodium sulfate and concentrated to give the title compound (61) (1.25 g, quant) that was used directly in the next step without further purification.

### Example 2: Preparation of tert-butyl bis(2-oxoethyl)carbamate (62)

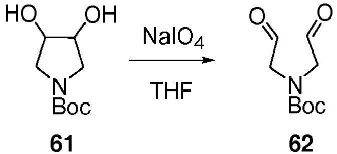

tert-Butyl 3,4-dihydroxypyrrolidine-1-carboxylate (61, 1.25 g, 6.1 mmol) was dissolved in THF (35 mL) and water (15 mL) and then treated with sodium periodate (1.97 g, 9.2 mmol). After 30 min the solids were removed by filtration and the reaction mixture was extracted with ethyl acetate (3 x 20 mL). The combined organics were dried with sodium sulfate and concentrated to give the title compound (62) as an oil (1.2 g, 99%) that was used directly in the next step without further purification.

### Example 3: Preparation of tert-butyl 4-(3-((1H-pyrrolo[2,3-b]pyridin-5-yl)oxy)-4-(methoxycarbonyl)phenyl)piperazine-1-carboxylate (15a)

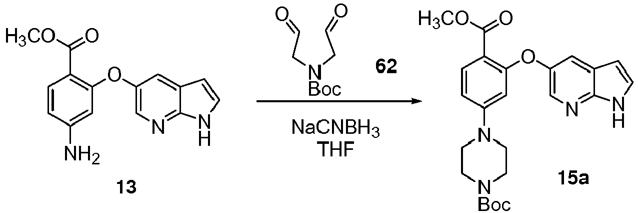

Methyl 2-((1H-pyrrolo[2,3-b]pyridin-5-yl)oxy)-4-aminobenzoate (13) (100 mg, 0.35 mmol) and tert-butyl bis(2-oxoethyl)carbamate (62) (149 mg, 0.74 mmol) were dissolved in methanol (1 mL) and acetic acid (0.5 mL) then cooled to 0 °C. Sodium cyanoborohydride (88.7 mg, 1.41 mmol) was added in 3 portions over 30 min. LCMS showed the title compound (15a) in 71% conversion.

### Example 4: Preparation of 2-((1H-pyrrolo[2,3-b]pyridin-5-y)oxy)-4-(piperazin-1-yl)benzonitrilebis hydrochloride (3b)

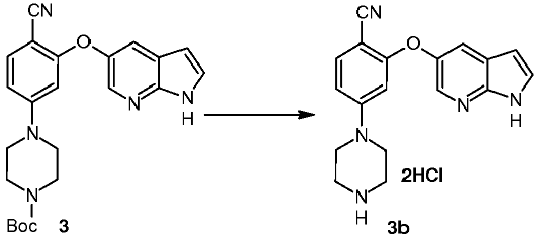

tert-Butyl 4-(3-((1 H-pyrrolo[2,3-b]pyridin-5-yl)oxy)-4-cyanophenyl)piperazine-1-carboxylate (**3**, 12.0 g, 28.6 mmol) was treated with 4N HCl/MeOH (prepared from AcCI/MeOH, 96 mL, 8 vol) and the resulting solution was warmed to 40-55 °C for 1-2 h during which time the reaction turned pale yellow followed by a cream color precipitate being observed. MTBE (48 mL, 4 vol) was added and the batch cooled to room temperature. The solids were collected, washed with MTBE/methanol (50/50 v/v, 2 x 24 mL), MTBE (2 x 24 mL) and conditioned for 1 h at room temperature to afford 2-((1 H-pyrrolo[2,3-b]pyridin-5-yl)oxy)-4-(piperazin-1-yl)benzonitrile bis-hydrochloride as a cream colored solid (**3b**, 10.9 g, 27.8 mmol, 97%). 1H NMR (500 MHz, DMSO-d6) δ 11.93 (s, 1H), 11.06 (br s, 1 H), 8.16 (d, J = 2.g Hz, 1 H), 7.81 (d, J = 2.5 Hz, 1 H), 7.67 (d, J = 8.85 Hz, 1 H), 7.58 (m, 1 H), 6.83 (dd, J = 8.9, 2.3 Hz, 1 H), 6.49 (dd, J = 3.4, 1.9 Hz, 1 H), 6.36 (d, J = 2.3 Hz, 1 H), 3.45-3.43 (m, 4H), 3.19-3.05 (m, 4H); 13C NMR (125 MHz, DMSO-d6) δ 161.3, 154.3, 145.6, 143.9, 134.7, 133.6, 128.8, 121.3, 120.8, 116.9, 109.6, 102.0, 100.7, 91.0, 43.5, 41.9; m/z: [M+H]+ = 320.0; mp = 235-238 °C (dec).

### Example 5a: Preparation of 2-((1H-pyrrolo[2,3-b]pyridin-5-yl)oxy)-4-(4-(tert-butoxycarbonyl)piperazin-1-yl)benzoic acid (4) from 3

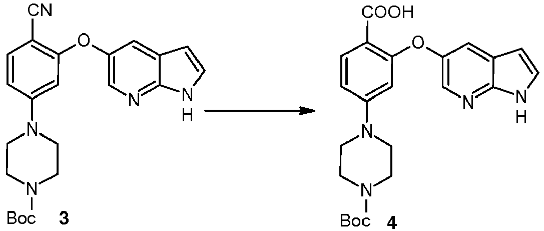

tert-Butyl 4-(3-((1H-pyrrolo[2,3-b]pyridin-5-yl)oxy)-4-cyanophenyl)piperazine-1-carboxylate (**3**, 2.0 g, 4.76 mmol) was treated with 4N HCl/MeOH (prepared from AcCI/MeOH, 20 mL, 20 vol) and the resulting dark purple solution was warmed to 40-55 °C for 1-2 h during which time the reaction turned pale yellow followed by a cream color precipitate being observed. 2-Butanol (20 mL, 10 vol) was added and the reaction was concentrated by atmospheric distillation until 20-22 mL of distillate was collected. A second charge of 2-butanol (20 mL, 10 vol) was added, the batch reheated to 80-90 °C and sodium hydroxide (flake, 2.48 g, 61.98 mmol, 13 equiv) was added. The batch was stirred with vigorous boiling for 24-42 h after which time the batch was cooled to room temperature and MTBE (60 mL, 30 vol) was added. The organic layer was removed and discarded and the aqueous layer was washed with isopropyl acetate (60 mL, 30 vol). The organic layer was removed and discarded and to the aqueous layer was then added isopropyl acetate (60 mL, 30 vol), 2-butanol (8 mL, 4 vol) and di-tert-butyl dicarbonate (1.04 g, 4.76 mmol, 1.0 equiv). The reaction mixture heated to 35-45 °C for 2-18 h, cooled to room temperature and the. pH adjusted to 2-4.2 with citric acid (2M, aqueous, 15 mL). Heptane (25 mL, 12 vol) was then added and the resulting mixture stirred for 5-30 min. As much as possible of the aqueous layer was removed, keeping the interfacial solids with the upper organic layer. The solids were collected on a Buchner funnel and rinsed with MTBE (2 x 5 mL) and conditioned for 10-60 min to give the title compound as an off-white solid (4, 1.75 g, 3.99 mmol, 84% isolated yield). 1 H NMR (500 MHz, DMSO-d6) δ 12.22 (s, 1 H), 11.59 (s, 1 H), 7.99 (d, J = 2.7 Hz, 1 H), 7.78 (d, J = 8.9 Hz, 1 H), 7.46 (t, J = 2.9 Hz, 1 H), 7.40 (d, J = 2.6 Hz, 1 H), 6.78 (dd, J = 8.9, 2.4 Hz, 1 H), 6.42 (d, J = 2.3, 1 H), 6.37-6.36 (m, 1 H), 3.39-3.37 (m, 4H), 3.32-3.18 (m, 4H), 1.39 (s, 9H); 13C NMR (125 MHz, DMSO-d6) δ 165.8, 158.3, 154.5, 153.8, 148.5, 144.9, 134.5, 133.4, 127.3, 119.7, 115.9, 111.9, 109.3, 105.3, 99.8, 79.0, 46.7, 42.1 (br), 27.9; m/z: [M+H]+ = 439.0; mp = 210-213 °C (dec).

### Example 5b: Alternate Procedure using isolated 3b bis-hydrochloride salt

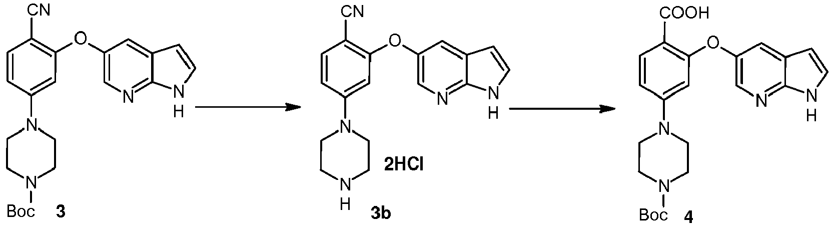

tert-Butyl 4-(3-((1H-pyrrolo[2,3-b]pyridin-5-yl)oxy)-4-cyanophenyl)piperazine-1-carboxylate (**3**, 6.0 g, 14.3 mmol) was treated with 4N HCl/MeOH (prepared from AcCI/MeOH, 45 mL, 7.5 vol) and the resulting dark purple solution was warmed to 40-55 °C for 1-2 h during which time the reaction turned pale yellow followed by a cream color precipitate being observed. The batch was cooled to 0-5 °C, the solid was collected, washed with methanol (2 x 6 mL), MTBE (2 x 10 mL) and conditioned for 1 h at room temperature to afford 2-((1H-pyrrolo[2,3-b]pyridin-5-yl)oxy)-4-(piperazin-1-yl)benzonitrile bis-hydrochloride as a cream colored solid (3b, 5.45 g, 13.9 mmol, 97%). Crude **3b** was charged to a reactor, 2-butanol (240 mL, 40 vol) was added, the batch heated to 80-90 °C and sodium hydroxide (flakes, 2.48 g, 61.98 mmol, 13 equiv) was added. The batch was stirred with vigorous boiling for 24-42 h after which time the batch was cooled to room temperature and MTBE (60 mL, 30 vol) was added. The organic layer was removed and discarded and the aqueous layer was washed with isopropyl acetate (60 mL, 30 vol). The organic layer was removed and discarded and to the aqueous layer was added isopropyl acetate (60 mL, 30 vol), 2-butanol (24 mL, 4 vol) and di-tert-butyl dicarbonate (1.04 g, 4.76 mmol, 1.0 equiv). The reaction mixture heated to 35-45 °C for 2-18 h, cooled to room temperature and the pH adjusted to 4-4.8 with aqueous potassium hydrogensulfate (2M, aqueous). The organic layer was removed, washed with water (60 mL), dried with anhydrous sodium sulfate, filtered and concentrated to give the title compound as a cream colored solid (**4**, 1.75 g, 3.99 mmol, 84% isolated yield).

### Example 6: Preparation of 2-((1H-pyrrolo[2,3-b]pyridin-5-yl)oxy)-4-(4-(tert-butoxycarbonyl)piperazin-1-yl)benzoic acid (4) using isolated 3b

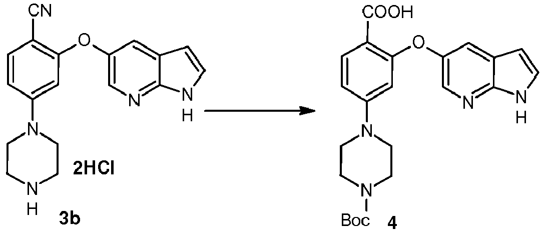

2-((1H-pyrrolo[2,3-b]pyridin-5-yl)oxy)-4-(piperazin-1-yl)benzonitrilebis hydrochloride (**3b**, 5.21 g, 13.28 mmol) and 2-butanol (200 mL, 37 vol) were charged to a reactor, the suspension heated to 80-90 °C and sodium hydroxide (flake, 2.48 g, 61.98 mmol, 13 equiv) was added. The batch was stirred with vigorous boiling for 24-42 h after which time the batch was cooled to room temperature and MTBE (100 mL, 19 vol) and water (100 mL, 19 vol) were added. The organic layer was removed and discarded and the aqueous layer was washed with isopropyl acetate (100 mL, 19 vol). The organic layer was removed and discarded. The aqueous layer was added back to the reactor followed by isopropyl acetate (100 mL, 19 vol), methanol (30 mL, 5.8 vol) and di-tert-butyl dicarbonate (2.89 g, 13.28 mmol, 1.0 equiv). The reaction mixture stirred at 18-40 °C for 2-18 h, set to room temperature and the pH adjusted to 2-4.2 with citric acid (2M, aqueous, 30 mL). Heptane (52 mL, 10 vol) was then added and the resulting mixture stirred for 5-30 min. As much as possible of the aqueous layer was removed, keeping the interfacial solids with the upper organic layer. The solids were collected on a Buchner funnel and rinsed with MTBE (2 x 2 mL) and conditioned for 10-60 min to give the title compound as an off-white solid (**4**, 4.29 g, 9.75 mmol, 74% isolated yield).

### Example 7: Preparation of tert-butyl 4-(3-((1H-pyrrolo[2,3-b]pyridin-5-yl)oxy)-4-(((3-nitro-4-(((tetrahydro-2H-pyran-4-yl)methyl)amino)phenyl)sulfonyl)carbamoyl) phenyl)piperazine-1-carboxylate (5)

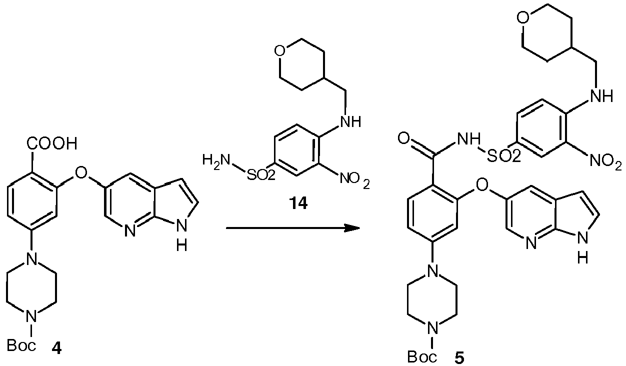

To a suspension of 2-((1H-pyrrolo[2,3-b]pyridin-5-yl)oxy)-4-(4-(tert-butoxycarbonyl)piperazin-1-yl)benzoic acid (**4**, 300 mg, 0.68 mmol, 1.00 equiv) in DCM (7.5 mL) was added triethylamine (145 mg, 1.43 mmol, 2.10 equiv). The suspension was stirred until it formed a solution then 3-nitro-4-(((tetrahydro-2H-pyran-4-yl)methyl)amino)benzenesulfonamide (**14**, 205 mg, 0.65 mmol, 0.95 equiv), EDAC (168 mg, 0.87 mmol, 1.3 equiv) and DMAP (105 mg, 1.25 equiv) were added and the reaction was stirred for 15-20 h at 30 °C. If the reaction was not complete by HPLC, additional portions of triethylamine, EDAC and DMAP were added in the same ratios as before until the reaction was complete. Quench by the addition of water (4.95 mL) followed by 2M citric acid solution (4.95 mL), IPAC (7.5 mL) and MTBE (3 mL). Stir for 5 min then add seed crystals (2 mg) and stir for 2-3 hr. Collect the crystals by vacuum filtration and wash with water (3 mL) and MTBE (5 mL) to give the title compound as a bright yellow solid (**5**, 324 mg, 0.44 mmol, 68% isolated yield). 1H NMR (500 MHz, DMSO-d6) δ 11.7 (s, 1 H), 11.5 (s, 1 H), 8.59 (t, J = 1.0 Hz, 1 H), 8.56 (s, 1 H), 8.05 (d, J = 2.6 Hz, 1H), 7.81 (dd, J = 9.2, 2.1, 1H), 7.53 (m, 3H), 7.12 (d, J = 9.3 Hz, 1H), 6.72 (dd, J = 9.0, 2.3 Hz, 1 H), 6.39 (dd, J = 3.4, 1.5 Hz, 1 H), 6.25 (d, J = 2.1 Hz, 1 H), 3.85 (dd, J = 11.3, 2.9 Hz, 2H), 3.35-3.20 (m, 9H), 3.15-3.12 (m, 4H), 1.92-1.78 (m, 1H), 1.63-1.60 (m, 1H), 1.37 (s, 9H), 1.27-1.24 (m, 2H); 13C NMR (125 MHz, DMSO-d6) δ 163.3, 157.7, 154.5, 153.6, 147.3, 146.4, 145.3, 135.1, 133.8, 132.0, 129.5, 127.8, 124.3, 119.7, 117.7, 114.9, 112.3, 108.9, 102.3, 99.9, 78.9, 66.5, 54.8, 47.9, 46.3, 43.2 (br), 33.8, 30.1, 27.9, 25.4; m/z: [M+H]+ = 736.0; mp = 167-170 °C (dec).

### Example 8: Preparation of 2-((1H-pyrrolo[2,3-b]pyridin-5-yl)oxy)-N-((3-nitro-4-(((tetrahydro-2H-pyran-4-yl)methyl)amino)phenyl)sulfonyl)-4-(piperazin-1-yl)benzamide bishydrochloride (6)

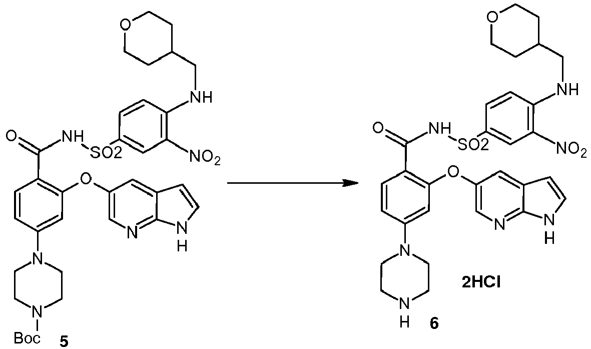

A solution of 4N HCl/MeOH/MeOAc was prepared by adding acetyl chloride (7 mL to methanol 93 mL at 0°C and stirring for no less than 1 h then warming to room temperature prior to use. A solution of tert-butyl 4-(3-((1H-pyrrolo[2,3-b]pyridin-5-yl)oxy)-4-(((3-nitro-4-(((tetrahydro-2H-pyran-4-yl)methyl)amino)phenyl)sulfonyl)carbamoyl)phenyl)piperazine-1-carboxylate (**5**, 950 mg, 1.29 mmol) in 4N HCl/MeOH/MeOAc (25 ml) was heated to 40-50 °C for 1-4 h during which time the desired product precipitated from the reaction mixture. The solids were collected by filtration, washing the reaction flask with recovered mother liquor to facilitate complete material collection. The cake was washed with methanol (2 x 2 mL) and MTBE (2 x 3 mL). The cake was conditioned for a further 30-60 min to give the desired product as a bright yellow crystalline solid (**6**, 880 mg, 1.24 mmol, 97% isolated yield). 1 H NMR (500 MHz, DMSO-d6) δ11.72 (s, 1 H), 11.68 (s, 1 H), 9.19 (brs, 1 H), 8.61 (t, J = 5.8 Hz, 1 H), 8.56 (d, J = 2.3 Hz, 1 H), 8.06 (d, J = 2.6 Hz, 1 H), 7.81 (dd, J = 9.2, 2.2 Hz, 1H), 7.56-7.51 (m, 3H), 7.12 (d, J = 9.4 Hz, 1H), 6.78 (dd, J = 8.9, 2.3 Hz, 1 H), 6.40 (dd, J = 3.4, 1.9 Hz, 1 H), 6.33 (d, J = 2.3 Hz, 1 H), 3.85 (dd, J = 11.2, 2.9 Hz, 2H), 3.76-3.66 (m, 4H), 3.35-3.24 (m, 4H), 3.09-3.07 (m, 4H), 1.91-1.87 (m, 1H), 1.62-1.60 (m, 2H), 1.31-1.22 (m, 2H); 13C NMR (125 MHz, DMSO-d6) δ163.5, 157.3, 153.9, 147.3, 146.4, 143.2, 133.7, 132.8, 132.0, 129.5, 128.6, 127.7, 124.3, 121.2, 120.1, 115.1, 113.6, 109.4, 103.4, 100.5, 66.5, 48.6, 47.9, 43.7, 33.8, 30.1, 26.7; m/z: [M+H]+ = 636.0; mp = 272-275 °C (dec).

### Example 9: Preparation of 2-((1H-pyrrolo[2,3-b]pyridin-5-yl)oxy)-4-(4-((4'-chloro-5,5-dimethyl-3,4,5,6-tetrahydro-[1,1'-biphenyl]-2-yl)methyl)piperazin-1-yl)-N-((3-nitro-4-(((tetrahydro-2H-pyran-4-yl)methyl)amino)phenyl)sulfonyl)benzamide (Venetoclax)

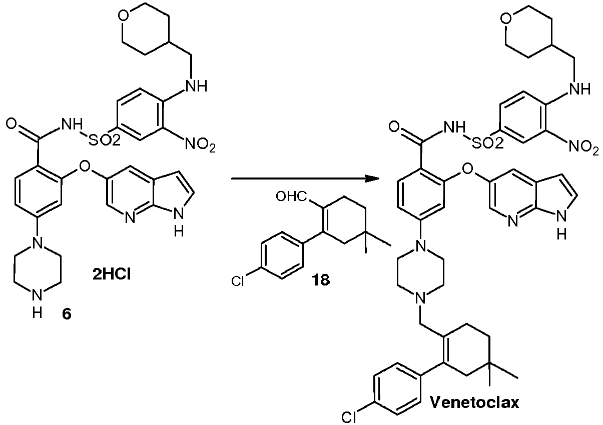

To a suspension of 2-((1H-pyrrolo[2,3-b]pyridin-5-yl)oxy)-N-((3-nitro-4-(((tetrahydro-2H-pyran-4-yl)methyl)amino)phenyl)sulfonyl)-4-(piperazin-1-yl)benzamide bis hydrochloride (**6**, 460 mg, 0.65 mmol, 1.0 equiv) in DCM (4.4 mL) and THF (4.6 mL) was added diisopropyl ethylamine (0.34 mL, 1.95 mmol, 3.0 equiv) to resulting in a homogeneous reaction mixture. After stirring for 5 min, 4'-chloro-5,5-dimethyl-3,4,5,6-tetrahydro-[1,1'-biphenyl]-2-carbaldehyde (18, 193 mg, 0.778 mmol, 1.2 equiv) was added and the reaction mixture stirred for 10 min then sodium triacetoxyborohydride (275 mg, 1.29 mmol, 2 equiv) was added in one portion and the reaction mixture stirred at 25-40 °C for 2-22 h. The reaction mixture was treated with 10% aqueous citric acid (10 mL), DCM (5 mL) and MeOH (2 mL). The aqueous layer was back extracted with isopropyl acetate (15 mL) and the combined organics were washed with saturated aqueous sodium bicarbonate (10 mL) and dried with anhydrous sodium sulfate, filtered and concentrated to dryness to give an orange foam (600 mg). The crude material was purified by chromatography on silica gel (Combiflash Gold 80 g column, 100% DCM to 5% MeOH/DCM). Clean fractions were combined and concentrated to dryness under vacuum to obtain the title compound as a bright yellow solid (Venetoclax, 650 mg, 0.75 mmol, 83% isolated yield).

### Example 10: Preparation of 2-((1H-pyrrolo[2,3-b]pyridin-5-yl)oxy)-4-(piperazin-1-yl)benzonitrile (40)

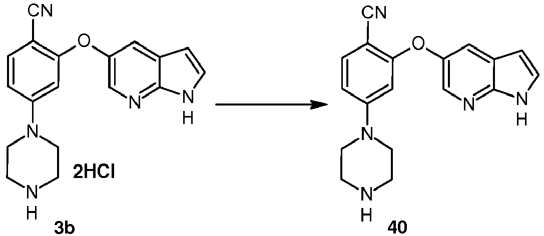

To a stirred suspension of 2-((1H-pyrrolo[2,3-b]pyridin-5-yl)oxy)-4-(piperazin-1-yl)benzonitrile bishydrochloride (**3b**, 1.00 g, 2.55 mmol) in isopropyl acetate (20 mL) was added saturated aqueous sodium bicarbonate (20 mL). The resulting gummy mixture was diluted with THF (10 mL) with no change in solubility. DCM (30 mL) was added which facilitated dissolution of the gummy matter. Allowed the phases to separate and kept the rag layer with the organic phase. The aqueous phase was further extracted with DCM (15 mL) A and the combined organic fractions were passed through a plug of diatomaceous earth to remove residual water. The organics were further dried with anhydrous sodium sulfate, filtered and concentrated under reduced pressure to afford the desired product as a pale yellow foam (800 mg, 98%). ¹H NMR (500 MHz, DMSO-d₆) δ 11.78 (s, 1 H), 8.09 (d, J = 2.7 Hz, 1 H), 7.73 (d, J = 2.6 Hz, 1 H), 7.56 (dd, J - 8.7, 4.8 Hz, 2H), 7.33 (dd, J = 6.7, 1.8 Hz, 2H), 7.02 (dd, J = 6.6, 1.8 Hz, 2H), 6.71 (m, 1 H), 6.45 (m, 1 H), 6.20 (d, J = 2.2 Hz, 1 H), 3.10-3.08 (m, 4H), 2.71 (s, 2H), 2.50 (m, 2H), 2.15-2.12 (m, 4H), 1.94 (s, 2H), 1.36 (t, J = 6.5 Hz, 2H), 0.92 (2, 6H). ¹³C NMR (125 MHz, DMSO-d₆) δ166.5, 159.6, 155.5, 154.5, 147.9, 147.8, 145.3, 138.1, 135.1, 128.5, 123.9, 120.3, 109.5, 109.2, 104.3, 102.4, 84.6, 80.3, 46.9, 42.1 (br); ESI MS m/z 320 [M+H]+.

### Example 11: Preparation of 2-((1H-pyrrolo[2,3-b]pyridin-5-yl)oxy)-4-(4-((4'-chloro-5,5-dimethyl-3,4,5,6-tetrahydro-[1,1'-biphenyl]-2-yl)methyl)piperazin-1-yl)benzonitrile (41)

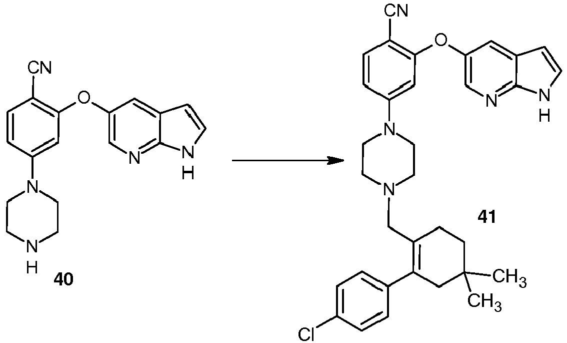

To a suspension of 2-((1H-pyrrolo[2,3-b]pyridin-5-yl)oxy)-4-(piperazin-1-yl)benzonitrile (**40,** 500 mg, 0.1.56 mmol, 1.0 equiv) in THF (10 mL, 20 vol) was added 4'-chloro-5,5-dimethyl-3,4,5,6-tetrahydro-[1,1'-biphenyl]-2-carbaldehyde (18, 428 mg, 1.72 mmol, 1.1 equiv) followed by an additional rinse of THF (5 mL, 10 vol). Sodium triacetoxyborohydride (611 mg, 3.12 mmol, 2 equiv) was added in one portion and the reaction mixture stirred at 18-28 °C for 2-22 h. The reaction mixture diluted with isopropylacetate (10 mL, 20 vol) and washed with 10% aqueous sodium hydroxide (10 mL, 20 vol). The aqueous layer was discarded and the organic layer was washed with saturated aqueous sodium chloride. The organics were concentrated to dryness under reduced pressure to give a light yellow foam (1.0 g). The crude material was purified by crystallization from hot acetonitrile (10 mL). The white solid product was collected after cooling to room temperature, the resulting cake was washed with pre-cooled (5 C) acetonitrile (2 x 2.5 mL) and conditioned for 1 hr. The product was dried at 50 C under high vacuum to give the desired product as a white crystalline solid (41, 636 mg, 1.16 mmol, 74% isolated yield). ¹H NMR (500 MHz, DMSO-d₆) δ 11.78 (s, 1H), 8.09 (d, J = 2.7 Hz, 1 H), 7.73 (d, J = 2.6 Hz, 1 H), 7.56 (dd, J = 8.7, 4.8 Hz, 2H), 7.33 (dd, J = 6.7, 1.8 Hz, 2H), 7.02 (dd, J = 6.6, 1.8 Hz, 2H), 6.71 (m, 1 H), 6.45 (m, 1 H), 6.20 (d, J = 2.2 Hz, 1 H), 3.10-3.08 (m, 4H), 2.71 (s, 2H), 2.50 (m, 2H), 2.15-2.11 (m, 4H), 1.94 (s, 2H), 1.36 (t, J = 6.5 Hz, 2H), 0.92 (s, 6H). 13C NMR (125 MHz, DMSO-d6) δ 161.4, 154.7, 145.7, 145.6, 141.9, 135.4, 134.0, 130.7, 129.9, 128.9, 128.0, 127.9, 126.1, 119.9, 118.7, 117.2, 108.8, 100.8, 100.1, 89.5, 59.6, 51.9, 46.3, 46.2, 39.9, 39.8, 34.7, 28.7, 27.8,25.1; ESI MS m/z 552 [M+H]+.

### Example 12: Preparation 2-((1H-pyrrolo[2,3-b]pyridin-5-yl)oxy)-4-(4-((4'-chloro-5,5-dimethyl-3,4,5,6-tetrahydro-[1,1'-biphenyl]-2-yl)methyl)piperazin-1-yl)benzoic acid hydrochloride (42)

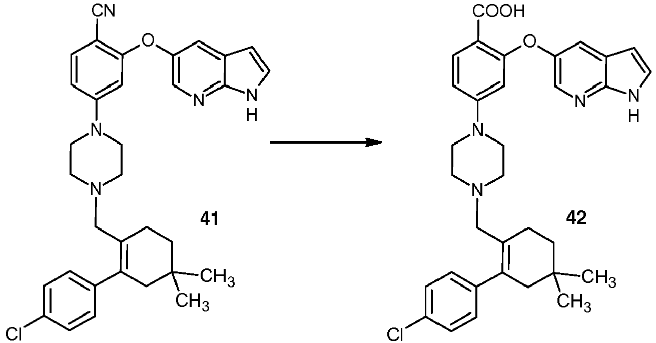

A biphasic solution of 2-((1H-pyrrolo[2,3-b]pyridin-5-yl)oxy)-4-(4-((4'-chloro-5,5-dimethyl-3,4,5,6-tetrahydro-[1,1'-biphenyl]-2-yl)methyl)piperazin-1-yl)benzonitrile (**41**, 1.08 g, 1.95 mmol) ethanol (10.8 mL), water (5.44 mL) and 50% sodium hydroxide in water (10.8 mL, 135 mmol) was heated to reflux for 48 h with vigorous stirring. The reaction was cooled to 18-25 °C, the biphasic mixture was allowed to settle and the aqueous layer was removed and discarded. 2-Methyl tetrahydrofuran (10.8 mL) and water (10.8 mL) were added and the batch was agitated for 5 min before letting the layers separate and removing the aqueous layer. Water (10 mL) was added and the batch pH adjusted to 6.5-7.5 by the addition of aqueous HCl (2M, 1.0-1.5 mL) which was followed by additional aqueous HCl (2M, 1.97 mL, 3.94 mmol, 2 equiv). Saturated aqueous sodium chloride (6 mL) was added and the aqueous layer was removed. Water (10 mL) was added and the desired product began to crystallize in the organic layer. The aqueous layer was removed and the product was collected by filtration. The filter cake was washed with MTBE (5 mL), conditioned for 30-60 min then dried under high-vacuum at 50-55 oC for 24-48 h to give the title compound as a white solid (0.98 g, 82%). 1H NMR (500 MHz, DMSO-d6) δ 12.31 (brs, 1H), 11.62 (s, 1H), 9.93 (brs, 1 H), 7.98 (d, J = 2.6 Hz, 1 H), 7.77 (d, J = 8.9 Hz, 1 H), 7.48 (t, J = 3.0 Hz, 1 H), 7.41-7.39 (m, 3H), 7.10 (d, J = 8.3 Hz, 2H), 6.76 (dd, J = 9.0, 2.4 Hz, 1H), 6.40 (d, J = 2.0 Hz, 1 H), 6.37 (m, 1 H), 3.71 (d, J = 12.0 Hz, 2H), 3.58-3.57 (m, 2H), 3.28 (d, J = 11.9 Hz, 2H), 3.20 (t, J = 8.5 Hz, 2H), 2.81-2.70 (m, 2H), 2.29 (brs, 2H), 2.20 (brs, 2H), 1.47 (t, J = 6.1 Hz, 2H), 0.95 (s, 6H). 13C NMR (125 MHz, DMSO-d6) δ; ESI MS m/z 571 [M+H]+.

### Example 13: Preparation of 2-((1H-pyrrolo[2,3-b]pyridin-5-yl)oxy)-4-(4-((4'-chloro-5,5-dimethyl-3,4,5,6-tetrahydro-[1,1'-biphenyl]-2-yl)methyl)piperazin-1-yi)-N-((3-nitro-4-(((tetrahydro-2H-pyran-4-yl)methyl)amino)phenyl)sulfonyl)benzamide (Venetoclax)

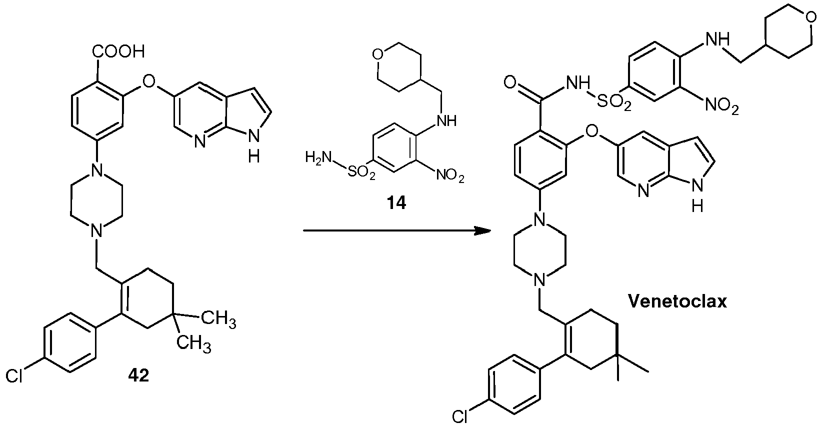

2-((1H-pyrrolo[2,3-b]pyridin-5-yl)oxy)-4-(4-((4'-chloro-5,5-dimethyl-3,4,5,6-tetrahydro-[1,1'-biphenyl]-2-yl)methyl)piperazin-1-yl)benzoic acid hydrochloride (**42**, 50 mg, 0.082 mmol) was suspended in DCM (1.25 mL), treated with triethylamine (0.035 mL, 0.251 mmol, 3.0 equiv) and stirred until a homogeneous solution was obtained. 3-Nitro-4-(((tetrahydro-2H-pyran-4-yl)methyl)amino)benzenesulfonamide (24 mg, 0.076 mmol, 0.93 equiv) and DMAP (12 mg, 0.098 mmol, 1.2 equiv) were added followed by EDAC (20 mg, 0.104 mmol, 1.3 equiv) and the reaction was stirred at 18-25 oC for 48 h. HPLC analysis (UV at 250-260 nm) showed the title compound (7) in 81% conversion (AUC).

## Claims

1. A process for preparing a compound of formula (I) or a salt or solvate thereof 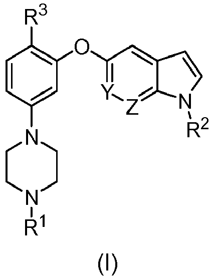 wherein
R¹ is selected from H, amino protecting group and -CH₂-R⁷;
R² is selected from H and amino protecting group;
R³ is selected from CN and COOR, wherein R is selected from H, C₁-C₆ alkyl, (C₆-C₁₀)aryl(C₁-C₆)alkyl, C₃-C₇ cycloalkyl and C₆-C₁₀ aryl;
R⁷ is selected from: 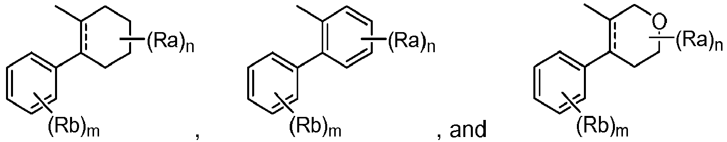 wherein
each Ra is independently selected from OH, CN, halogen and C₁₋C₆ alkyl,
each Rb is independently selected from OH, CN, halogen and C₁₋C₆ alkyl,
m and n are independently selected from 0, 1, 2 or 3, and
--- is a single or double bond;
Y is selected from CH and N; and
Z is selected from CH and N;
which comprises reductive amination of a compound of formula (II) or a salt or solvate thereof 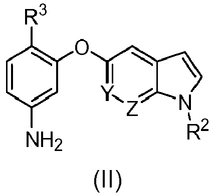 wherein R², R³, Y and Z are as defined above, by:
- reaction with a compound of formula (III) or a salt or solvate thereof 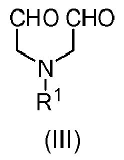 wherein R¹ is selected from H, amino protecting group and -CH₂R⁷, to provide a compound of formula (I) or a salt or solvate thereof;
or
- reaction with a compound of formula (IV) or a salt or solvate thereof 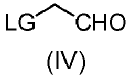
wherein LG is a leaving group, to provide a compound of formula (V) or a salt or solvate thereof 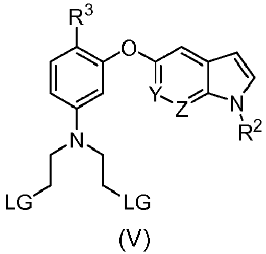 wherein R², R³, Y and Z are as defined above and LG is a leaving group,
and
reaction of a compound of formula (V), or a salt or solvate thereof, with a compound of formula (VI) or a salt or solvate thereof
R¹-NH₂ (VI)
wherein R¹ is selected from H, amino protecting group and -CH₂R⁷, to provide a compound of formula (I) or a salt or solvate thereof.

2. Process according to claim 1, wherein the compound of formula (I), or a salt or solvate thereof, is prepared by reacting a compound of formula (II) or a salt or solvate thereof 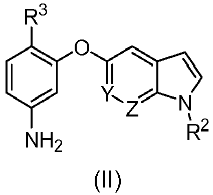 wherein
R² is selected from H and amino protecting group;
R³ is selected from CN and COOR, wherein R is selected from H, C₁-C₆ alkyl, (C₆-C₁₀)aryl(C₁-C₆)alkyl, C₃-C₇ cycloalkyl and C₆-C₁₀ aryl;
Y is selected from CH and N; and
Z is selected from CH and N;
with a compound of formula (III) or a salt or solvate thereof 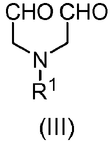 wherein R¹ is selected from H, amino protecting group and -CH₂-R⁷.

3. Process according to claim 1, wherein the compound of formula (I), or a salt or solvate thereof, is prepared by:
reaction of a compound of formula (II) or a salt or solvate thereof 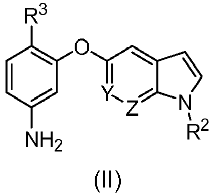 wherein
R² is selected from H and amino protecting group;
R³ is selected from CN and COOR, wherein R is selected from H, C₁₋C₆ alkyl, (C₆-C₁₀)aryl(C₁C₆)alkyl, C₃-C₇ cycloalkyl and C₆-C₁₀ aryl;
Y is selected from CH and N; and
Z is selected from CH and N;
with a compound of formula (IV) or a salt or solvate thereof 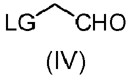 wherein LG is a leaving group, to provide a compound of formula (V) or a salt or solvate thereof 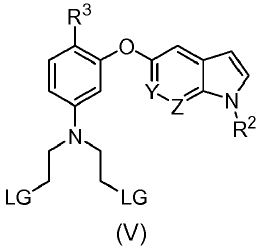 wherein R², R³, Y and Z are as defined above and LG is a leaving group, and
reaction of a compound of formula (V), or a salt or solvate thereof, with a compound of formula (VI) or a salt or solvate thereof
R¹-NH₂ (VI)
wherein R¹ is selected from H, amino protecting group and -CH₂-R⁷.

4. Process according to any one of claims 1 to 3, wherein Y is CH and Z is N.

5. Process according to any one of claims 1 to 4, wherein R¹ is an amino protecting group.

6. Process according to any one of claims 1 to 5, wherein R² is H.

7. Process according to any one of claims 1 to 6, wherein R³ is COOR, wherein R is selected from H, C₁-C₆ alkyl, (C₆-C₁₀)aryl(C₁-C₆)alkyl, C₃-C₇ cycloalkyl and C₆-C₁₀ aryl.

8. Process according to any one of claims 1 to 7, wherein the reaction of a compound of formula (II), or a salt or solvate thereof, with an aldehyde of formula (III) or (IV), or a salt or solvate thereof, is performed in the presence of a reductive agent selected from a borohydride, such as NaBH₄, NaCNBH₃, NaBH(OAc)₃, Bu₄NBH₃CN, LiCNBH₃ or 5-ethyl-2-methylpyridine borane.

9. Process according to any one of claims 1 to 8, which further comprises converting a compound of formula (I), or a salt or solvate thereof, into a compound of formula (A) or a salt or solvate thereof 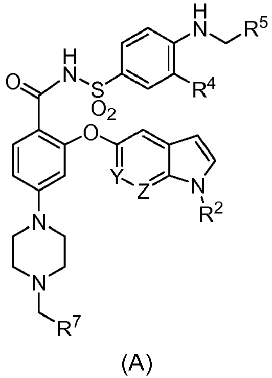 wherein
R² is selected from H and amino protecting group;
Y is selected from CH and N;
Z is selected from CH and N;
R⁴ is selected from H, NO₂, CN, CF₃ and halogen;
R⁵ is selected from H, C₃-C₇ cycloalkyl, and 5- to 10-membered heterocyclyl, optionally substituted by OH, CN, halogen, C₁-C₆ alkyl and C₃-C₇ cycloalkyl; and
R⁷ is selected from: 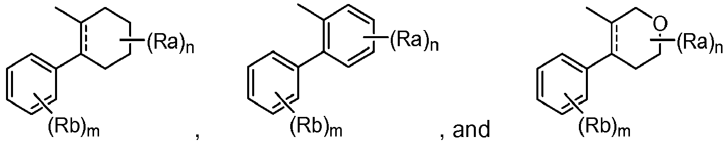 wherein
each Ra is independently selected from OH, CN, halogen and C₁₋C₆ alkyl,
each Rb is independently selected from OH, CN, halogen and C₁₋C₆ alkyl,
m and n are independently selected from 0, 1, 2 or 3, and
--- is a single or double bond.

10. Process according to claim 9, which comprises converting a compound of formula (I) wherein R¹ is selected from H and amino protecting group, or a salt or solvate thereof, into the compound of formula (A), or a salt or solvate thereof, by a process comprising:
(a) if R³ in the compound of formula (I) is CN or COOR, wherein R is selected from C₁-C₆ alkyl, (C₆-C₁₀)aryl(C₁-C₆)alkyl, C₃-C₇ cycloalkyl and C₆-C₁₀ aryl, converting it into a compound of formula (Ia) or a salt or solvate thereof 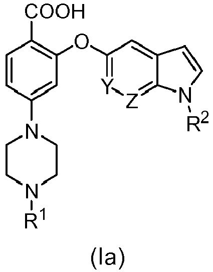 wherein
R¹ is selected from H and amino protecting group;
R² is selected from H and amino protecting group;
Y is selected from CH and N; and
Z is selected from CH and N;
(b) reacting a compound of formula (Ia), or a salt or solvate thereof, with a compound of formula (VII) or a salt or solvate thereof 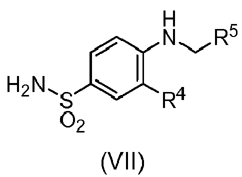 wherein
R⁴ is selected from H, NO₂, CN, CF₃ and halogen; and
R⁵ is selected from H, C₃-C₇ cycloalkyl, and 5- to 10-membered heterocyclyl, optionally substituted by OH, CN, halogen, C₁-C₆ alkyl and C₃-C₇ cycloalkyl;
to provide a compound of formula (VIII) or a salt or solvate thereof 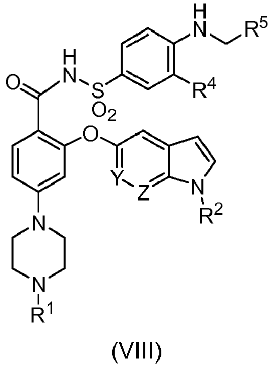 wherein R¹, R², R⁴, R⁵, Y and Z are as defined above;
(c) if R¹ is an amino protecting group, deprotecting it to provide a compound of formula (VIII) wherein R¹ is H, or a salt or solvate thereof; and
(d) reacting a compound of formula (VIII) wherein R¹ is H, or a salt or solvate thereof, with a compound of formula (IX) or with a compound of formula (X) R⁷-CHO (IX) 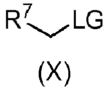 wherein
LG is a leaving group; and
R⁷ is selected from: 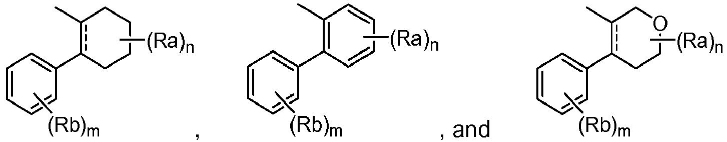 wherein
each Ra is independently selected from OH, CN, halogen and C₁-C₆ alkyl,
each Rb is independently selected from OH, CN, halogen and C₁-C₆ alkyl,
m and n are independently selected from 0, 1, 2 or 3, and
--- is a single or double bond;
to provide a compound of formula (A), or a salt or solvate thereof, as defined in claim 9.

11. Process according to any claim 9, which comprises converting a compound of formula (I) wherein R¹ is selected from H and amino protecting group, or a salt or solvate thereof, into the compound of formula (A), or a salt or solvate thereof, by a process comprising:
(a') if R¹ in the compound of formula (I) is an amino protecting group, deprotecting it to provide a compound of formula (Ib), or a salt or solvate thereof, 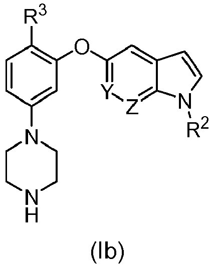 wherein
R² is selected from H and amino protecting group;
R³ is selected from CN and COOR, wherein R is selected from H, C₁-C₆ alkyl, (C₆-C₁₀)aryl(C₁-C₆)alkyl, C₃-C₇ cycloalkyl and C₆-C₁₀ aryl;
Y is selected from CH and N; and
Z is selected from CH and N;
(b') reacting a compound of formula (Ib), or a salt or solvate thereof, with a compound of formula (IX) or with a compound of formula (X)
R⁷-CHO (IX) 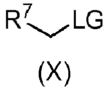
wherein
LG is a leaving group; and
R⁷ is selected from: 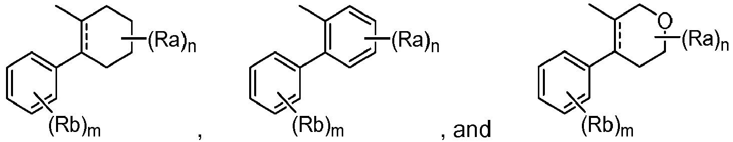 wherein
each Ra is independently selected from OH, CN, halogen and C₁-C₆ alkyl,
each Rb is independently selected from OH, CN, halogen and C₁-C₆ alkyl,
m and n are independently selected from 0, 1, 2 or 3, and
--- is a single or double bond;
to provide a compound of formula (Ic) or a salt or solvate thereof 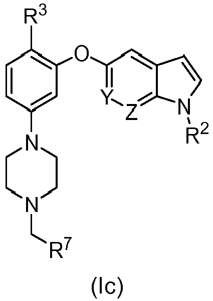 wherein R², R³, R⁷, Y and Z are as defined above;
(c') if R³ in the compound of formula (Ic), or a salt or solvate thereof, is CN or COOR, wherein R is selected from C₁-C₆ alkyl, (C₆-C₁₀)aryl(C₁-C₆)alkyl, C₃-C₇ cycloalkyl and C₆-C₁₀ aryl, converting it into a compound of formula (Id) or a salt or solvate thereof 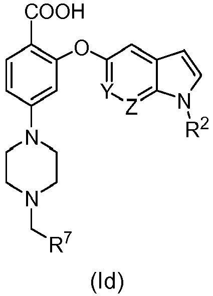 wherein R², R⁷, Y and Z are as defined above; and
(d') reacting a compound of formula (Id), or a salt or solvate thereof, with a compound of formula (VII) or a salt or solvate thereof 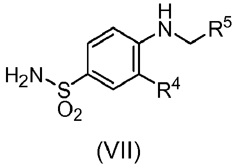 wherein
R⁴ is selected from H, NO₂, CN, CF₃ and halogen; and
R⁵ is selected from H, C₃-C₇ cycloalkyl, and 5- to 10-membered heterocyclyl, optionally substituted by OH, CN, halogen, C₁-C₆ alkyl and C₃-C₇ cycloalkyl;
to provide a compound of formula (A), or a salt or solvate thereof, as defined in claim 9.

12. Process according to claim 9, which comprises converting a compound of formula (I) wherein R¹ is -CH₂R⁷, or a salt or solvate thereof, into the compound of formula (A), or a salt or solvate thereof, by a process comprising:
(a") if R³ in the compound of formula (I) is CN or COOR, wherein R is selected from C₁₋C₆ alkyl, (C₆-C₁₀)aryl(C₁C₆)alkyl, C₃-C₇ cycloalkyl and C₆-C₁₀ aryl, converting it into a compound of formula (Id) or a salt or solvate thereof 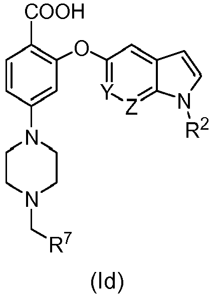 wherein
R² is selected from H and amino protecting group;
Y is selected from CH and N;
Z is selected from CH and N; and
R⁷ is selected from: 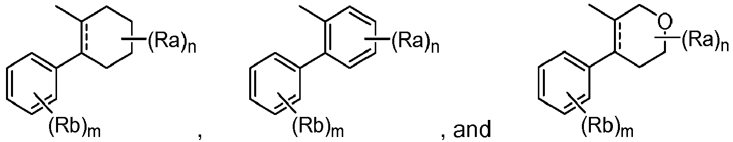 wherein
each Ra is independently selected from OH, CN, halogen and C₁-C₆ alkyl,
each Rb is independently selected from OH, CN, halogen and C₁-C₆ alkyl,
m and n are independently selected from 0, 1, 2 or 3, and
--- is a single or double bond; and
(b") reacting a compound of formula (Id), or a salt or solvate thereof, with a compound of formula (VII) or a salt or solvate thereof 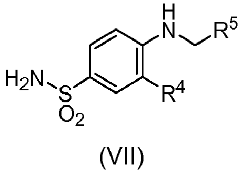 wherein
R⁴ is selected from H, NO₂, CN, CF₃ and halogen; and
R⁵ is selected from H, C₃-C₇ cycloalkyl, and 5- to 10-membered heterocyclyl, optionally substituted by OH, CN, halogen, C₁-C₆ alkyl and C₃-C₇ cycloalkyl;
to provide a compound of formula (A), or a salt or solvate thereof, as defined in claim 9.

13. Process according to any one of claims 9 to 12, wherein the compound of formula (A) is Venetoclax or a salt or solvate thereof.

14. A compound of formula (V), or a salt or solvate thereof 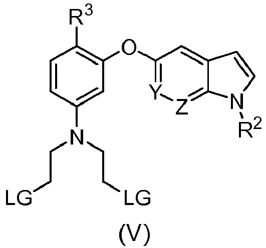 wherein
R² is selected from H and amino protecting group;
R³ is selected from CN and COOR, wherein R is selected from H, C₁-C₆ alkyl, (C₆-C₁₀)aryl(C₁-C₆)alkyl, C₃-C₇ cycloalkyl and C₆-C₁₀ aryl;
Y is selected from CH and N;
Z is selected from CH and N; and
LG is a leaving group.

15. A compound according to claim 14 selected from 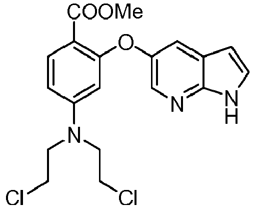 and a salt or solvate thereof.
